(19) Europäisches Patentamt

European Patent Office

Office européen des brevets

(11) **EP 4 712 096 A1**

(12) **EUROPEAN PATENT APPLICATION**

(43) Date of publication:
**18.03.2026  Bulletin 2026/12**

(21) Application number: **25201081.4**

(22) Date of filing: **09.09.2025**

(51) International Patent Classification (IPC):
**G16H 40/63** (2018.01)        **G16H 10/60** (2018.01)

(52) Cooperative Patent Classification (CPC):
**G16H 10/60; G16H 40/63**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:  **11.09.2024  US 202418830908**

(71) Applicant: **Tortus AI Ltd**
**London E1 0SG (GB)**

(72) Inventor: **TAN, Christopher**
**London, E3 4EH (GB)**

(74) Representative: **Mathys & Squire**
**The Shard**
**32 London Bridge Street**
**London SE1 9SG (GB)**

(54) **TABULAR POLICY ACTION MODELS FOR CLINICAL APPLICATIONS**

(57)    In some aspects, the present disclosure provides a computer-implemented system comprising: a digital processing device comprising: at least one processor, an operating system configured to perform executable instructions, a memory, and a computer program including instructions executable by the digital processing device to perform a computer task by a user expressing to the computer-implemented system, and wherein the computer-implemented system is configured to perform the task by generating and executing a computer-executable program.

```
         101                              102
  ┌──────────────────┐        ┌──────────────────┐
  │                  │        │                  │
  │  Determine Task  │        │  Determine State │
  │                  │        │                  │
  └──────────────────┘        └──────────────────┘
           │                           │
           ▼                           ▼
       103 ┌───────────────────────────┐
           │                           │
           │      Determine Action     │
           │                           │
           └───────────────────────────┘
                       │
       104             ▼
           ┌───────────────────────────┐
           │                           │
           │       Perform Action      │
           │                           │
           └───────────────────────────┘
```

*FIG. 1*

**Description**

**CROSS REFERENCE**

This application claims the benefit of the U.S. Application No. 18/830,908 filed September 11, 2024, which is incorporated herein by reference in its entirety.

**BACKGROUND**

An action model can be used to perform actions on a computer.

In some aspects, the present disclosure provides a method of performing a task by expressing to a computer configured to perform the task by generating and executing a computer-executable program that is based on (a) determining the task from the expressing and (b) obtaining digital information displayed by the computer.

In some aspects, the present disclosure provides a computer-implemented method of performing a task based on a user expression, comprising generating and executing a computer-executable program based on (a) determining the task from the expression and (b) obtaining digital information displayed by a computer.

In some embodiments, the determining the task comprises receiving an expression produced by the expressing through a receiver.

In some embodiments, the determining the task comprises processing the expression using a neural network. In some embodiments, the neural network comprises a large language model. In some embodiments, the neural network comprises a natural language model.

In some embodiments, the determining the task comprises generating a transcript of an expression produced by the expressing. In some embodiments, the determining the task comprises generating a prediction of the task based on the expression or the transcript.

In some embodiments, the obtaining the digital information comprises obtaining an image of a graphical user interface (GUI). In some embodiments, the image is shown on a display in electronic communication with the computer. In some embodiments, the image comprises a screenshot of the display. In some embodiments, the image comprises pixels. In some embodiments, the image comprises a vector graphic. In some embodiments, the image is a digital representation of information shown on the display.

In some embodiments, the obtaining the digital information comprises detecting a user interface element (UIE) in the image. In some embodiments, the obtaining the digital information comprises generating a sequence representation of the image. In some embodiments, the sequence representation comprises text. In some embodiments, the sequence representation comprises bits. In some embodiments, the sequence representation comprises a property of the UIE. In some embodiments, the property comprise a class of the UIE, a bounding box of the UIE, a position of the UIE, a text in the UIE, or any combination thereof.

In some embodiments, the generating and executing the computer-executable program is based on determining a state of the computer. In some embodiments, the determining the state comprises matching the digital information to a reference in a set of reference digital information. In some embodiments, the set of reference digital information comprises a set of images of GUIs. In some embodiments, the set of reference digital information comprises a set of screenshots.

In some embodiments, the matching comprises using a neural network. In some embodiments, the neural network comprises a convolutional network.

In some embodiments, the computer-executable program comprises instructions for performing an action. In some embodiments, the generating and executing the computer-executable program is based on determining the action. In some embodiments, the determining the action comprises matching the task and the state to the action in a set of possible actions. In some embodiments, the set of possible actions are tabulated. In some embodiments, the set of possible action are indexed by possible tasks and possible states.

In some embodiments, the action identifies a target UIE. In some embodiments, the target UIE is identified among a set of UIEs based on a spatial characteristic of the target UIE in relation to other user interface elements. In some embodiments, the spatial characteristic comprises distance between the target UIE and the other UIEs in the set. In some embodiments, the distance comprises a relative normalized pixel distance between the target UIE and the other UIEs in the set. In some embodiments, the relative normalized pixel distance is stored in table for retrieval.

In some embodiments, the action comprises typing. In some embodiments, the action comprises typing a character, a word, a phrase, a sentence, or a paragraph. In some embodiments, the action comprises a key press. In some embodiments, the action comprises a combination key press.

In some embodiments, the action comprises moving a cursor. In some embodiments, the action comprises moving the cursor to a target position. In some embodiments, the action comprises clicking. In some embodiments, the action comprises clicking a target position. In some embodiments, the target position has a target UIE.

In some embodiments, the action comprises waiting. In some embodiments, the action comprises waiting for a time period.

In some embodiments, the action comprises a large language model call.

In some embodiments, the action comprises optical character recognition.

In some embodiments, the action comprises determining a state of the computer.

In some embodiments, the action comprises determining that the task is complete.

In some embodiments, the action comprises a loop. In some embodiments, the loop performs the action on an iterable data type. In some embodiments, the loop performs an iterable action, an iterable task, or both.

In some embodiments, the state of the digital information is determined on a virtual machine. In some embodiments, the computer-executable program is executed on a virtual machine.

In some embodiments, the task comprises dictating. In some embodiments, the task comprises transcribing. In some embodiments, the dictating comprises dictating not every spoken word. In some embodiments, the dictating comprises dictating a subset of the spoken words. In some embodiments, the dictating comprises dictating while improving grammar.

In some embodiments, the task comprises searching. In some embodiments, the task comprises searching a database. In some embodiments, the task comprises searching a search engine.

In some embodiments, the task comprises opening a document.

In some embodiments, the task comprises ordering an item. In some embodiments, the item is a medical item. In some embodiments, the item is a prescription, an X-ray, a test, a discharge instruction, an examination, or any combination thereof.

In some embodiments, the task comprises taking notes.

In some embodiments, the task comprises writing an email or a text message.

In some embodiments, the task comprises sending a voice recording.

In some embodiments, the task comprises calling for a medical professional.

In some embodiments, the task comprises interacting with medical software.

In some embodiments, the expressing is speaking. In some embodiments, an expression produced by the expressing is speech. In some embodiments, the expressing is writing. In some embodiments, an expression produced by the expressing is written. In some embodiments, the expressing is typing. In some embodiments, an expression produced by the expressing is typed. In some embodiments, the expressing is verbal, non-verbal, or both. In some embodiments, the expressing is directed to a human. In some embodiments, the expressing is directed to the computer. In some embodiments, the expressing is done by a medical professional. In some embodiments, the medical professional is a doctor, a nurse, a therapist, or an EMT.

In some embodiments, the expressing is done by a subject. In some embodiments, the subject is a patient. In some embodiments, the subject is paralyzed. In some embodiments, the subject is blind.

In some embodiments, the computer comprises a smartphone, a tablet, a laptop, a desktop, a server, or a cloud. In some embodiments, the computer comprises a mouse, a keyboard, and a display. In some embodiments, the receiver comprises a mic. In some embodiments, the receiver comprises a keyboard. In some embodiments, the receiver comprises a camera.

In some embodiments, the generating the computer-executable program comprises generating computer readable instructions. In some embodiments, the generating the computer-executable program comprises generating human readable instructions. In some embodiments, the generating the computer-executable program comprises generating instructions in the computer's memory. In some embodiments, the generating the computer-executable program comprises generating instructions in the computer's storage. In some embodiments, the executing the computer-executable program comprises loading the computer-executable program from storage into memory.

In some embodiments, the method further comprises providing a message to a user when the task cannot be determined or performed. In some embodiments, the method further comprises providing a message to a user when the expression cannot be understood. In some embodiments, the method further comprises providing a message to a user when the action cannot be determined or performed. In some embodiments, the method further comprises providing a message when the task is complete. In some embodiments, the message is displayed on a screen by the computer to the user. In some embodiments, the message is played as sound by the computer to the user.

In some aspects, the present disclosure provides a computer-implemented system comprising: a digital processing device comprising: at least one processor, an operating system configured to perform executable instructions, a memory, and a computer program including instructions executable by the digital processing device to perform a computer task by a user expressing to the computer-implemented system, and wherein the computer-implemented system is configured to perform the task by generating and executing a computer-executable program, comprising: (a) determining the task from the expressing; and (b) obtaining digital information displayed by the computer.

In some aspects, the present disclosure provides a computer-implemented system comprising: a digital processing device comprising: at least one processor, an operating system configured to perform executable instructions, a memory, and a computer program including instructions executable by the digital processing device to perform a computer task based on a user expression, and wherein the computer-implemented system is configured to perform the task by generating and executing a computer-executable program, comprising: (a) determining the task from the expression; and (b) obtaining digital information displayed by the computer.

In some embodiments, the determining the task comprises receiving an expression produced by the expressing through a receiver. In some embodiments, the determining the task comprises processing the expression using a neural network. In some embodiments, the neural network comprises a large language model. In some embodiments, the neural network comprises a natural language model.

In some embodiments, the determining the task comprises generating a transcript of an expression produced by the expressing. In some embodiments, the determining the task comprises generating a prediction of the task based on the expression or the transcript.

In some embodiments, the obtaining the digital information comprises obtaining an image of a graphical user interface (GUI). In some embodiments, the image is shown on a display in electronic communication with the computer. In some embodiments, the image comprises a screenshot of the display. In some embodiments, the image comprises pixels. In some embodiments, the image comprises a vector graphic. In some embodiments, the image is a digital representation of information shown on the display.

In some embodiments, the obtaining the digital information comprises detecting a user interface element (UIE) in the image. In some embodiments, the obtaining the digital information comprises generating a sequence representation of the image. In some embodiments, the sequence representation comprises text. In some embodiments, the sequence representation comprises bits. In some embodiments, the sequence representation comprises a property of the UIE. In some embodiments, the property comprise a class of the UIE, a bounding box of the UIE, a position of the UIE, a text in the UIE, or any combination thereof.

In some embodiments, the generating and executing the computer-executable program is based on determining a state of the computer. In some embodiments, the determining the state comprises matching the digital information to a reference in a set of reference digital information. In some embodiments, the set of reference digital information comprises a set of images of GUIs. In some embodiments, the set of reference digital information comprises a set of screenshots.

In some embodiments, the matching comprises using a neural network. In some embodiments, the neural network comprises a convolutional network. In some embodiments, the computer-executable program comprises instructions for performing an action.

In some embodiments, the generating and executing the computer-executable program is based on determining the action. In some embodiments, the determining the action comprises matching the task and the state to the action in a set of possible actions. In some embodiments, the set of possible actions are tabulated. In some embodiments, the set of possible action are indexed by possible tasks and possible states.

In some embodiments, the action identifies a target UIE. In some embodiments, the target UIE is identified among a set of UIEs based on a spatial characteristic of the target UIE in relation to other user interface elements. In some embodiments, the spatial characteristic comprises distance between the target UIE and the other UIEs in the set. In some embodiments, the distance comprises a relative normalized pixel distance between the target UIE and the other UIEs in the set. In some embodiments, the relative normalized pixel distance is stored in table for retrieval.

In some embodiments, the action comprises typing. In some embodiments, the action comprises typing a character, a word, a phrase, a sentence, or a paragraph. In some embodiments, the action comprises a key press. In some embodiments, the action comprises a combination key press. In some embodiments, the action comprises moving a cursor. In some embodiments, the action comprises moving the cursor to a target position. In some embodiments, the action comprises clicking. In some embodiments, the action comprises clicking a target position. In some embodiments, the target position has a target UIE.

In some embodiments, the action comprises waiting. In some embodiments, the action comprises waiting for a time period.

In some embodiments, the action comprises a large language model call.

In some embodiments, the action comprises optical character recognition.

In some embodiments, the action comprises determining a state of the computer.

In some embodiments, the action comprises determining that the task is complete.

In some embodiments, the action comprises a loop. In some embodiments, the loop performs the action on an iterable data type. In some embodiments, the loop performs an iterable action, an iterable task, or both.

In some embodiments, the state of the digital information is determined on a virtual machine. In some embodiments, the computer-executable program is executed on a virtual machine.

In some embodiments, the task comprises dictating. In some embodiments, the task comprises transcribing. In some embodiments, the dictating comprises dictating not every spoken word. In some embodiments, the dictating comprises dictating a subset of the spoken words. In some embodiments, the dictating comprises dictating while improving grammar.

In some embodiments, the task comprises searching. In some embodiments, the task comprises searching a database. In some embodiments, the task comprises searching a search engine.

In some embodiments, the task comprises opening a document.

In some embodiments, the task comprises ordering an item. In some embodiments, the item is a medical item. In some embodiments, the item is a prescription, an X-ray, a test, a discharge instruction, an examination, or any combination thereof.

In some embodiments, the task comprises taking notes.

In some embodiments, the task comprises writing an email or a text message.

In some embodiments, the task comprises sending a voice recording.

In some embodiments, the task comprises calling for a medical professional.

In some embodiments, the task comprises interacting with medical software.

In some embodiments, the expressing is speaking. In some embodiments, an expression produced by the expressing is speech. In some embodiments, the expressing is writing. In some embodiments, an expression produced by the expressing is written. In some embodiments, the expressing is typing. In some embodiments, an expression produced by the expressing is typed. In some embodiments, the expressing is verbal, non-verbal, or both.

In some embodiments, the expressing is directed to a human. In some embodiments, the expressing is directed to the computer.

In some embodiments, the expressing is done by a medical professional. In some embodiments, the medical professional is a doctor, a nurse, a therapist, or an EMT.

In some embodiments, the expressing is done by a subject. In some embodiments, the subject is a patient. In some embodiments, the subject is paralyzed. In some embodiments, the subject is blind.

In some embodiments, the computer comprises a smartphone, a tablet, a laptop, a desktop, a server, or a cloud. In some embodiments, the computer comprises a mouse, a keyboard, and a display. In some embodiments, the receiver comprises a mic. In some embodiments, the receiver comprises a keyboard. In some embodiments, the receiver comprises a camera.

In some embodiments, the generating the computer-executable program comprises generating computer readable instructions. In some embodiments, the generating the computer-executable program comprises generating human readable instructions. In some embodiments, the generating the computer-executable program comprises generating instructions in the computer's memory. In some embodiments, the generating the computer-executable program comprises generating instructions in the computer's storage. In some embodiments, the executing the computer-executable program comprises loading the computer-executable program from storage into memory.

In some embodiments, the computer program including the instructions further comprises providing a message to a user when the task cannot be determined or performed. In some embodiments, the computer program including the instructions further comprises providing a message to a user when the expression cannot be understood. In some embodiments, the computer program including the instructions further comprises providing a message to a user when the action cannot be determined or performed. In some embodiments, the computer program including the instructions further comprises providing a message when the task is complete. In some embodiments, the message is displayed on a screen by the computer to the user. In some embodiments, the message is played as sound by the computer to the user.

In some embodiments, the system further comprises a display. In some embodiments, the system further comprises a receiver. In some embodiments, the system further comprises a storage. In some embodiments, the system is a cloud-computing system. In some embodiments, the system comprises a virtual machine.

In some aspects, the present disclosure provides a method of performing a click by expressing to a computer configured to perform the click by generating and executing a computer-executable program that is based on distinguishing the correct click target that comprises the same text as an incorrect click target.

In some aspects, the present disclosure provides a computer-implemented method of performing a click based on a user expression, comprising generating and executing a computer-executable program based on distinguishing the correct click target that comprises the same text as an incorrect click target.

In some aspects, the present disclosure provides a computer-implemented system comprising: a digital processing device comprising: at least one processor, an operating system configured to perform executable instructions, a memory, and a computer program including instructions executable by the digital processing device to perform a click by a user expressing to the computer-implemented system, and wherein the computer-implemented system is configured to perform the click by generating and executing a computer-executable program based on distinguishing the correct click target that comprises the same text as an incorrect click target.

In some aspects, the present disclosure provides a computer-implemented system comprising: a digital processing device comprising: at least one processor, an operating system configured to perform executable instructions, a memory, and a computer program including instructions executable by the digital processing device to perform a computer task by a user expressing an expression to the computer-implemented system, and wherein the computer-implemented system is configured to perform the task by generating and executing a computer-executable program, comprising: (a) determining the computer task from the expression, by: (i) receiving the user's expression through a receiver in electronic communication with the computer-implemented system; and (ii) processing the user's expression using a large language model to generate a prediction of the computer task that is desired by the user based on the user's expression, wherein the computer task involves performing an action using an electronic medical record application, and wherein the prediction is non-deterministic; (b) determining a state of digital information displayed on a display in electronic communication with the computer-implemented system; (c) determining the action to be performed, by matching the computer task and the state to the action in a set of possible actions, wherein the set of possible actions are tabulated and indexed by possible computer tasks and possible states, and wherein the matching is deterministic; and (d) generating and executing a computer-executable program that comprises instructions for performing the action, thereby performing the computer task.

In some embodiments, the determining the state of the digital information comprises obtaining an image of a graphical user interface (GUI). In some embodiments, the image comprises pixels. In some embodiments, the image is shown on a display in electronic communication with the computer. In some embodiments, the image is a digital representation of information shown on the display. In some embodiments, the determining the state of the digital information comprises detecting a user interface element (UIE) in the image.

In some embodiments, the obtaining the digital information comprises generating a sequence representation of the image. In some embodiments, the sequence representation comprises a property of the UIE, wherein the property comprises a class of the UIE, a bounding box of the UIE, a position of the UIE, a text in the UIE, or any combination thereof.

In some embodiments, the action identifies a target UIE. In some embodiments, the target UIE is identified among a set of UIEs based on a spatial characteristic of the target UIE in relation to other user interface elements. In some embodiments, the spatial characteristic comprises distance between the target UIE and the other UIEs in the set. In some embodiments, the distance comprises a relative normalized pixel distance between the target UIE and the other UIEs in the set. In some embodiments, the relative normalized pixel distance is stored in table for retrieval. In some embodiments, the action comprises clicking the target UIE. In some embodiments, the action comprises a combination key press.

In some embodiments, the action comprises a loop, wherein the loop performs the action on an iterable data type. In some embodiments, the task comprises dictating. In some embodiments, the task comprises searching. In some embodiments, the task comprises ordering an item.

In some embodiments, the computer task comprises interacting with graphical user interface elements. In some embodiments, the computer-executable program comprises instructions that are executable at a user-interface level. In some embodiments, the computer-executable program comprises instructions that are executable above a firmware level, an operating system level, or an application programming interface level.

In some aspects, the present disclosure provides a computer-implemented system comprising: a digital processing device comprising: at least one processor, an operating system configured to perform executable instructions, a memory, and a computer program including instructions executable by the digital processing device to perform a computer task by a user expressing an expression to the computer-implemented system, and wherein the computer-implemented system is configured to perform the task by generating and executing a computer-executable program, comprising: (a) determining the computer task from the expression, by: (i) receiving the user's expression through a receiver in electronic communication with the computer-implemented system; and (ii) processing the user's expression using a large language model to generate a prediction of the computer task that is desired by the user based on the user's expression, wherein the computer task involves performing an action using an electronic medical record application, and wherein the prediction is non-deterministic; (b) determining a state of digital information displayed on a display in electronic communication with the computer-implemented system, by: (i) obtaining an image of a graphical user interface (GUI) that is shown on the display; (ii) detecting a plurality of user interface elements (UIEs) in the image; (iii) generating a sequence representation of the image comprising a plurality of properties of the UIEs; and (iv) matching, using a neural network, the sequence representation to a reference in a set of reference digital information; (c) determining the action to be performed, by: (i) matching the computer task and the state to the action in a set of possible actions, wherein the set of possible actions are tabulated and indexed by possible computer tasks and possible states, and wherein the matching is deterministic; and (ii) identifying the target UIE among the plurality of UIEs in the digital information based on the action and a spatial characteristic of the target UIE in relation to other UIEs in the plurality, wherein the spatial characteristic comprises a relative normalized pixel distance between the target UIE and the other UIEs in the plurality; and (d) generating and executing a computer-executable program that comprises instructions for performing the action, thereby performing the computer task.

A computer-implemented system comprising: a digital processing device comprising: at least one processor, an operating system configured to perform executable instructions, a memory, and a computer program including instructions executable by the digital processing device to perform a computer task by a user expressing an expression to the computer-implemented system, and wherein the computer-implemented system is configured to perform the task by generating and executing a computer-executable program, comprising: (a) determining the computer task from the expression, by: (i) receiving the user's expression through a receiver in electronic communication with the computer-implemented system; and (ii) processing the user's expression using a large language model to generate a prediction of the computer task that is desired by the user based on the user's expression, wherein the computer task involves performing an action using an electronic medical record application, and wherein the prediction is non-deterministic; (b) determining a state of digital information displayed on a display in electronic communication with the computer-implemented system; (c) determining the action to be performed, by matching the computer task and the state to the action in a set of enumerated actions, wherein the set of enumerated actions are indexed by computer tasks and display states, and wherein the matching is deterministic; and (d) generating and executing a computer-executable program that comprises instructions for performing the action, thereby performing the computer task.

## INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

## BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

FIG. 1 illustrates a schematic of an action model, in accordance with some embodiments.

FIG. 2 illustrates a schematic of an action model, in accordance with some embodiments.

FIG. 3 illustrates a schematic of an action model, in accordance with some embodiments.

FIGS. 4A-4C show examples of tasks.

FIG. 5 shows an example of a GUI and features of the GUI that can be detected, in accordance with some embodiments.

FIG. 6 illustrates various levels of completeness of an action language, in accordance with some embodiments.

FIG. 7 illustrates a computer system, in accordance with some embodiments.

## DETAILED DESCRIPTION

In some aspects, the present disclosure provides systems and methods implementing an action model. Action model can use an artificial intelligence (AI) model to take actions in a computer environment of a user, in order to achieve a goal or complete a task. An action model can be a model that receives a variety of input types (e.g., text, audio, or video) and take an action.

For illustrative purposes, an action model can be contrasted against LLMs. LLMs can be models which receive and output only text. A user may use such an LLM to help organize a holiday. The LLM may offer a recommendation on where to go and what to do on the holiday, as well as providing a step-by-step plan of what actions to take on an internet browser to book flights and hotels. However, regardless of how accurate these steps might be, an LLM (solely by itself) may not actually book the flights or hotels on a user's behalf. This is because when an LLM's outputs are configured only to be text, it is not configured to take actions in a computer environment.

On the other hand, an action model may take control of a computer (e.g., by using a user's mouse and keyboard) and book the flights and hotels on the user's behalf. The action's model's policy - which could be a function that predicts what action the action model should take - can be parameterized using a neural network. A potential benefit of using a neural network to parameterize a policy is generalizability. A well generalizable model may be able to complete not just tasks that it has seen during training, but also completely unseen tasks. Thus, some action models comprise a policy network. The policy network can comprise a neural network. In some cases, the action model can be trained using reinforcement learning, behavioral cloning, both.

Parameterizing a policy with a neural network can lead to some drawbacks, however. Some of these drawbacks can make the action model difficult to apply in clinical applications. One drawback may be data inefficiency. For a toy model on mini simulated computer environment (MiniWob++; 165 x 220 pixels), training data comprising thousands of hours of expert demonstration footage may be made before training a neural network policy for an action model. Obtaining this huge volume of manually collected data can be extremely costly. Scaling the same approach to achieve human level performance on realistic tasks and computer screen sizes may require orders of magnitudes more data.

One drawback may be lack of interpretability. Deep learning models may have features which are in a "black-box," such as their policies that are parameterized or "spread" over millions or billions of parameters. These parameters individually may not be human-interpretable. This can be particularly undesirable for clinical applications since a lack of interpretability may make it difficult to troubleshoot or fix an action model. If the model performs an incorrect action, it can be difficult to know why it performed the incorrect action, and to fix it directly to prevent future problems.

One drawback may be rampancy, or "hallucinations." "Hallucinations" can refer to outputs of AI models that are "made up" or "untrue." For example, certain LLMs can exhibit hallucinations by stating a clearly untrue fact or producing a nonsensical statement / gibberish. This deficiency can be dangerous in a clinical setting, if for example, an action model hallucinates and assigns a treatment plan or prescribes medications that are not what a medical professional intended.

One drawback may be model regression. Model regression in machine learning can occur when a model's performance deteriorates over time, between different versions, or when it is trained on new data. For action models that are configured to perform multiple tasks, model regression can be a significant problem since when new data for a new task is introduced, training on the new data can deteriorate the model's performance on previously learned tasks. In practice, model regression can make it very difficult to ensure that model performance across all tasks of interests monotonically increases over time, over different versions, and with new data.

In some aspects, the present disclosure provides an action model comprising a tabular policy. A policy can refer to a function that maps states to actions. A tabular policy can refer to a policy that comprises a table to map states to actions. In a computer control setting, the state can include 1) an observation of the screen (e.g., the pixels), and 2) the user command. The action space can include actions that a human user can do on the computer, e.g., controlling the mouse and typing on the keyboard.

The policy can be denoted by $\pi$, states by s, and actions by $a$. For a deep learning action model, policy can be parameterized by neural network having $\Theta$ which can denote the set of model parameters. Then, the policy can be written as $\pi_\Theta (a \mid s)$.

For example, a user command can be "get me a cute cat picture from Google," and the screen state can be the home desktop screen. Then the action could be to click the mouse at a specified coordinate (x=1194, y=955), which corresponds to the location of the a web browser icon on the screen, which would then launch the web browser. This action can be expressed as:

$$\pi_\Theta(click(x = 1194, y = 955) \mid s = (\text{"get cat picture"}, home\ screen)) = 1$$

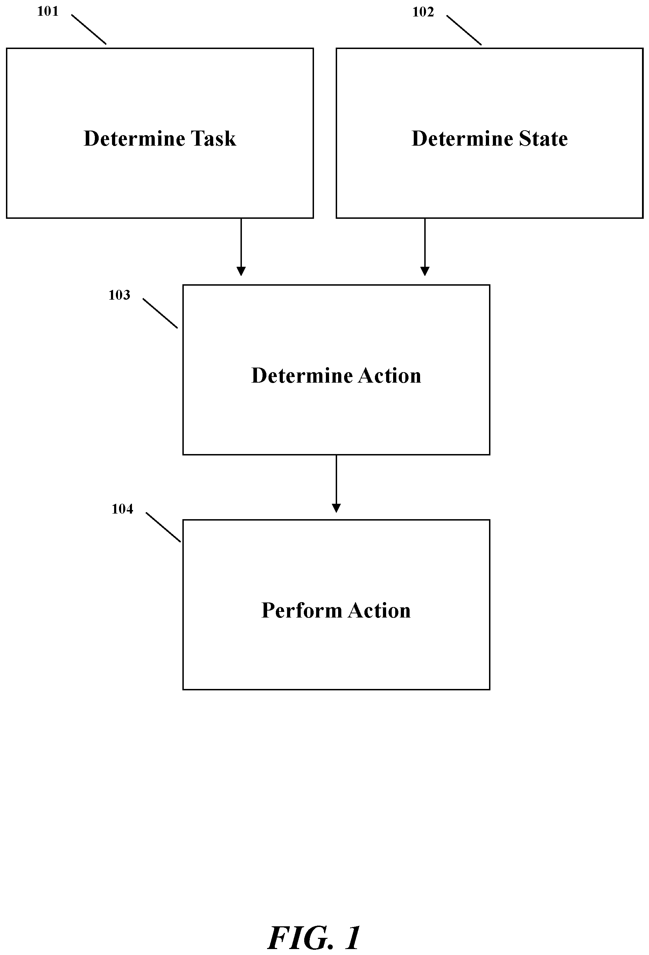

**FIG. 1** illustrates a schematic of an action model, in accordance with some embodiments. An action model can determine a task to perform **101,** determine a state of the computer 102, and based on the task and the state, determine **103** and perform **104** the action.

In a tabular policy, the policy $\pi$ can be made without neural network parameters $\Theta$. And so, the policy can be explicitly defined in a table (e.g., $\pi_{table}$). Evaluating the policy function for a specific state can be performed by looking up a table, rather than performing a forward pass of a neural network.

In contrast to a policy that is parameterized by a deep neural network, using a tabular policy can be more data efficient. A tabular policy may avoid using data in as large amount as is typically used to pre-train neural network based policies. A tabular policy may avoid neural network fine-tuning problems to learn new tasks in the policy. A tabular policy can be updated to one-shot any new task, when items in the tabular policy that corresponds to the new task has been added, e.g., an action sequence for completing the task. Moreover, the tabular policy can be updated to capture edge-cases or unexpected issues (e.g., if a random pop-up appears on the user interface) without requiring neural network fine-tuning for each update. In some cases, depending on the screen matching approach used, performance can be improved with more data (e.g., if perceptual hashing is used, adding more reference hashes can improve performance). Perceptual hashing can refer to the use of a fingerprinting algorithm that produces a snippet, hash, or fingerprint of various forms of multimedia. A perceptual hash can refer to a type of locality-sensitive hash, which is analogous if features of the multimedia are similar.

A tabular policy can be more interpretable than neural network policies. Because the tabular policy can be explicitly defined, by its own construction, the policy can be fully interpretable. Each state, action, state-action pair, and task can be investigated, and the model can be expected to behave deterministically and predictably based on the tabular policy.

A tabular policy can be designed to prevent hallucinations or generalization rampancy of actions. Unlike neural network policies, where controlling the generalization-rampancy tradeoff is often more art than science due to the policy's black box aspects, using a tabular policy that allows treating the task matching and the state matching as separate sub-problems can be individually adjusted to control for the model's generalization-rampancy tradeoff (e.g., by finely controlling or tuning the confidence threshold for each sub-problem such as determining the task and determining the state).

While the task matching and the state matching can be implementing using a neural network which can be non-deterministic (or probabilistic), the tabular policy can be implemented using a table (or equivalent) which can determine actions deterministically (and predictably). Because the possible actions that the action model can take can be explicitly defined and enumerated, an action model with a tabular policy can be restricted to taking actions which are hard-programmed into the action model, and be prevented from taking rampant (or unforeseeable) actions. Preventing the hallucination of actions and rampancy of action models can have a further effect of improving the accuracy of the actions taken.

A tabular policy can eliminate the phenomenon of model regression entirely. In contrast to neural network policies, where new task data causes a parametrization of the entire policy network thereby potentially negatively affecting its performance on previously stable tasks, a tabular policy can simply treat new task data as additional state-action pairs that get appended as new rows, leaving the already existing task policies unchanged.

An action model comprising a tabular policy can be lighter than with a neural network policy. As a result, the action model may be implemented on smaller devices, consuming less memory, and less computational resources. As a tabular policy action model can avoid having to train on an enormous dataset of expert demonstrations, the tabular policy action model can be made and used while consuming less computational resources.

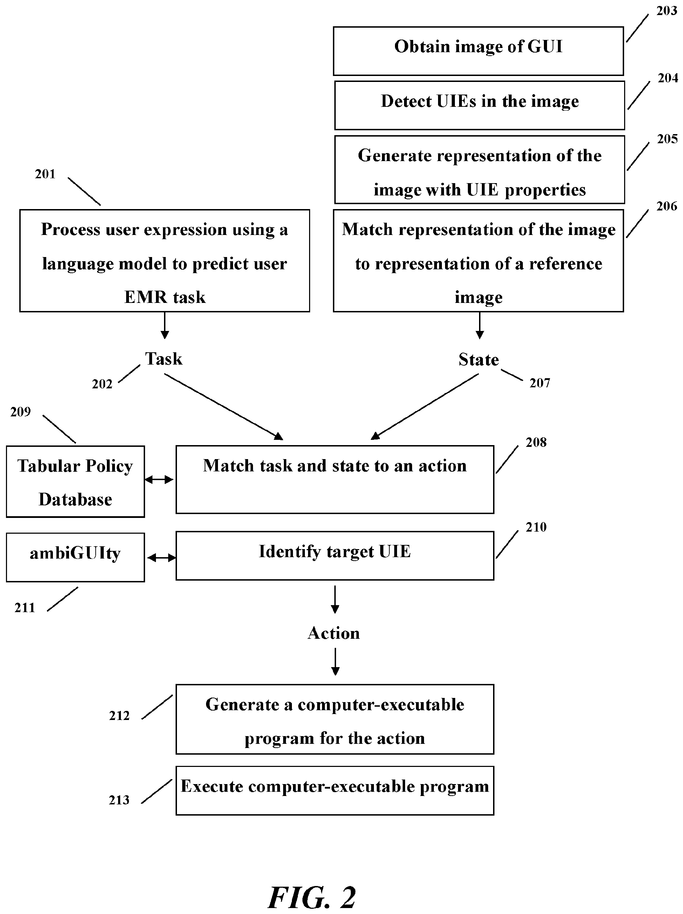

Accordingly, in some aspects, the present disclosure provides a computer-implemented system. **FIG. 2** illustrates a schematic of an action model, in accordance with some embodiments. The computer-implemented system can comprise a digital processing device. The digital processing device can comprise at least one processor, an operating system configured to perform executable instructions, a memory, and a computer program including instructions executable by the digital processing device. The instructions can be configured to perform a computer task by a user expressing to the computer-implemented system. The computer-implemented system can be configured to perform the task by generating and/or executing a computer-executable program.

The computer-executable program can be generated and/or executed by determining **201** the computer task **202** from the user expressing. The computer task can be determined by receiving the user's expression through a receiver in electronic communication with the computer-implemented system. The computer task can be determined by processing the user's expression using a large language model to generate a prediction of the computer task that is desired by the user based on the user's expression. The computer task can involve performing an action using an electronic medical record application. The prediction can be non-deterministic.

The computer-executable program can be generated and/or executed by determining a state of digital **207** information displayed on a display in electronic communication with the computer-implemented system. The state of digital information can be determined by obtaining **203** an image of a graphical user interface (GUI) that is shown on the display. The state of digital information can be determined by detecting **204** a plurality of user interface elements (UIEs) in the image. The state of digital information can be determined **205** by generating a sequence representation of the image comprising a plurality of properties of the UIEs. The state of digital information can be determined **206** by matching, using a neural network, the sequence representation to a reference in a set of reference digital information.

The computer-executable program can be generated and/or executed by determining the action to be performed. The action to be performed can be determined by matching the computer task and/or the state to the action in a set of possible (or a set of enumerated) actions **208.** The set of possible actions can be tabulated and indexed by possible computer tasks and possible states **209** (e.g., list of computer tasks and display states that are enumerated in the tabular policy). The matching can be deterministic. The action to be performed can be determined by identifying **210** the target UIE among the plurality of UIEs in the digital information. Identifying the target UIE can be based on the action and a spatial characteristic **211** of the target UIE in relation to other UIEs in the plurality. The spatial characteristic can comprise a relative normalized pixel distance between the target UIE and the other UIEs in the plurality. The computer-implemented system can be configured to perform the task by generating **212** and/or executing **213** the computer-executable program that comprises instructions for performing the action, thereby performing the computer task.

In some cases, the action to be performed can be determined using a neural network policy. Although neural network policy may have drawbacks for certain tasks especially in relation to high risk ones (e.g., ordering a prescription or surgery), it can still be used to perform certain benign tasks that the tabular policy does not explicitly encode for (e.g., looking up a picture of a cat, or a dog). In some cases, the neural network policy can be used to augment the prediction of a task even if it is a high risk task, but which can be accompanied by a warning message to alert a user to require human review.

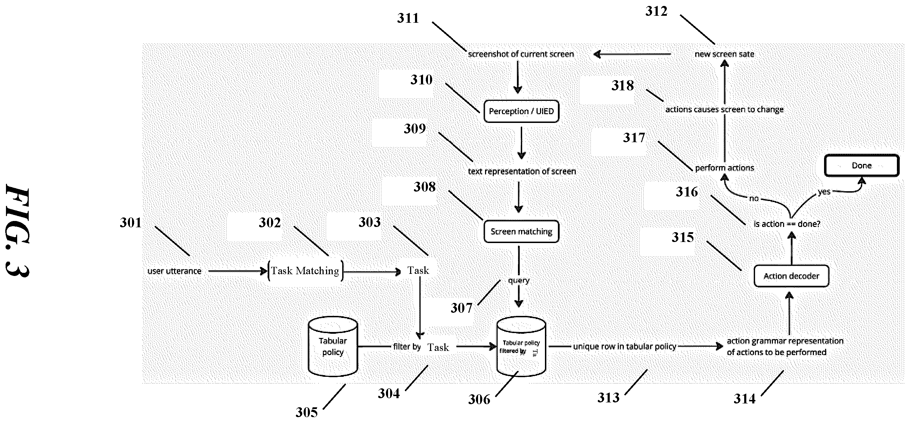

**FIG. 3** illustrates schematic of an action model, in accordance with some embodiments. In some aspects, the present disclosure provides a method of performing a task by a user expressing **301** to a computer configured to perform the task. In some cases, the user's expression can be matched **302** to a computer task **303.** A tabular policy can be filtered **304** based on the matched computer task. For example, elements in the tabular policy that involve the computer task can be retrieved without retrieving elements that do not involve the computer task. A filtered tabular policy **306** can receive a query **307** that is based on a current screen state **312** of a computer device. The query **307** can be generated by obtaining a screenshot **311** of the current screen state, processing **310** the screenshot to obtain a text representation of the screen **309,** and matching **308** that text representation to a database of textual screen representations.

A unique action **313** from the filtered tabular policy **306** can be selected based on the query. The action grammar representation **314** from the filtered tabular policy corresponding to the unique action **313** can be processed by an action decoder **315** to perform the action according to the action grammar representation **314.** The method can comprise confirming that the action according to the user's intent was executed completely **316.** If the action was not executed completely, the further actions **317** can be performed to cause the current screen state to change **313.** A different unique action can be obtained by generating a query **307** corresponding to the current screen state **312,** and selecting the action from the filtered tabular policy **306.**

In some aspects, the present disclosure provides a method of performing a task by expressing to a computer configured to perform the task by generating and/or executing a computer-executable program that is based on determining the task from the expressing, obtaining digital information displayed by the computer, or both. In some aspects, the present disclosure provides a computer-implemented method of performing a task based on a verbal command, comprising generating and/or executing a computer-executable program based on determining the task from the expressing, obtaining digital information displayed by the computer, or both.

In some aspects, the present disclosure provides a computer-implemented system. The computer-implemented system can comprise a digital processing device. The digital processing device can comprise at least one processor, an operating system configured to perform executable instructions, a memory, and a computer program including instructions executable by the digital processing device. The instructions can be configured to perform a computer task by a user expressing to the computer-implemented system. The instructions can be configured to perform a computer task based on a user expression. The computer-implemented system can be configured to perform the task by generating and/or executing a computer-executable program. The generating and/or executing the computer-executable program can be based on determining the task from the expressing, obtaining digital information displayed by the computer, or both.

In some aspects, the present disclosure provides a method of performing a click. The method can comprise expressing to a computer configured to perform the click. The computer can be configured to perform the click by generating and/or executing a computer-executable program. The computer-executable program can be based on distinguishing the correct click target that comprises the same text as an incorrect click target. In some aspects, the present disclosure provides a computer-implemented method of performing a click. The computer-implemented method can perform a click based on a verbal command. The computer-implemented method can generate and/or execute a computer-executable program based on distinguishing the correct click target that comprises the same text as an incorrect click target.

In some aspects, the present disclosure provides a computer program product comprising a computer-readable medium having computer-executable code encoded therein, the computer-executable code adapted to be executed to implement any one of the methods or computer-implemented methods disclosed herein. In some aspects, the present disclosure provides a non-transitory computer-readable storage media encoded with a computer program including instructions executable by one or more processors to perform any one of the methods or computer-implemented methods disclosed herein.

## TASK MATCHING

In some cases, a method of the present disclosure comprises determining a task. In some cases, a system of the present disclosure can be configured to determine a task. Determining a task can comprise task matching. Task matching can comprise using a text classification model takes as input a user expression (e.g., an utterance) as a string, and assigns it to a task among a set of known tasks. In some cases, the user expression can comprise audio (e.g., of the user's speech), a string (e.g., a text transcription of the user's expression), a video (e.g., the user's body language and/or facial expressions), or any combination thereof. The task can be the underlying purpose or goal of the user, and various expressions can express the task in a variety of different ways.

For example, there may be three possible tasks: (1) Write the note; (2) Send the note; and (3) Save the letter. Each of these three tasks, however, can be expressed in number of different ways. A user might say "create the note please" to mean the task "write the note," or they might say "help me send off note" to mean the task "send the note." Task matching can be used to map any given expression to a task.

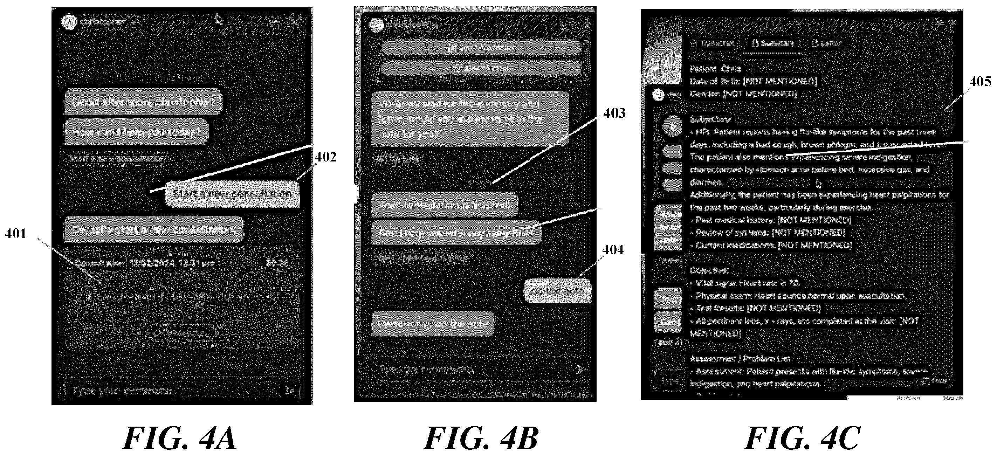

Another example of a task is to set up for a consultation between a medical professional and a subject. **FIG. 4A** shows an example of a graphical user interface for a user to interact with the action model. The action model can receive and transcribe the user's speech **401** into text, and then execute the task **402** of setting up for a new consultation. Starting a new consultation can comprise opening an electronic health record application, a note taking application, or any other preparation that the user specifies or is learned from the user's usage patterns.

Another example of a task is to automate a writing process for a medical professional. As shown in **FIG. 4B,** the action model can suggest **403** a task that the action model thinks it should take next. The user can instruct **404** the action model to take said action. The action can be writing a note based on the consultation.

Another example of a task is to summarize a consultation or a meeting for a medical professional. As shown in **FIG. 4C,** the action model can produce a summary **405** of the consultation based on the conversation between the medical professional and a subject.

In some cases, determining the task comprises receiving an expression through a receiver. In some cases, determining the task comprises processing the expression using a neural network. In some cases, the neural network comprises a large language model. In some cases, the neural network comprises a natural language model. In some cases, the determining the task comprises generating a transcript of the expression. In some cases, the determining the task comprises generating a prediction of the task based on the expression or the transcript.

In some cases, the task comprises dictating. In some cases, the task comprises transcribing. In some cases, the dictating comprises dictating not every spoken word. In some cases, the dictating comprises dictating a subset of the spoken words. In some cases, the dictating comprises dictating while improving grammar. Dictating and transcribing can be of a medical professional's spoken notes, a subject or a patient's complaints and issues, a discussion between medical professionals for a treatment plan, for example.

In some cases, the task comprises searching. In some cases, the task comprises searching a database. In some cases, the task comprises searching a search engine. Searching can be searching for one or a combination of symptoms exhibited by a subject or a patient, for reference images of a medical scan, a patient's medical history or family history, for example.

In some cases, the task comprises opening a document. The document can be a patient file, for example. In some cases, the task comprises ordering an item. In some cases, the item is a medical item. In some cases, the item is a prescription, an X-ray, a test, a discharge instruction, an examination, or any combination thereof.

In some cases, the task comprises taking notes. In some cases, the task comprises writing an email or a text message. In some cases, the task comprises sending a voice recording. In some cases, the task comprises calling for a medical professional. In some cases, the task comprises interacting with medical software.

## PERCEPTION

In some cases, a method of the present disclosure comprises perceiving a display. In some cases, a system of the present disclosure can be configured to perceive a display. The perception can comprise taking or obtaining a screenshot of the current display (e.g., a computer screen) and output a representation of the screen. The representation can be a structured representation. The representation can be a text representation. The representation can be generated using user interface element detection (UIED). The UIED can output a list of all UI elements on the screen, including their bounding boxes, classes, inner texts, or any combination thereof.

For example, a UIED can output for screen with NUI elements: [{id: 0, class: button, bounding_box: (1250, 50, 40, 50), inner_text: "Accept"}, {id: 1, class: text_field, bounding_box: (900, 750, 20, 30), inner_text: "First name" }, ..., {id: N-1, class: image, bounding_box: (400, 120, 100, 220), inner_text: None}].

In some cases, the obtaining the digital information comprises obtaining an image of a graphical user interface (GUI). In some cases, the image is shown on a display in electronic communication with the computer. In some cases, the image comprises a screenshot of the display. In some cases, the image comprises pixels. In some cases, the image comprises a vector graphic. In some cases, the image is a digital representation of information shown on the display. In some cases, the obtaining the digital information comprises detecting a user interface element (UIE) in the image. In some cases, the obtaining the digital information comprises generating a sequence representation of the image. In some cases, the sequence representation comprises text. In some cases, the sequence representation comprises bits. In some cases, the sequence representation comprises a property of the UIE. In some cases, the property comprise a class of the UIE, a bounding box of the UIE, a position of the UIE, a text in the UIE, or any combination thereof.

## SCREEN MATCHING

In some cases, a method of the present disclosure comprises determining a state. In some cases, a system of the present disclosure can be configured to determine a state. Determining a state can comprise matching a display to a set of known displays. In some cases, the matching can be a screen matching. The matching can be performed using an image classification model that matches an input screenshot image of a screen to screen in a set of known screens.

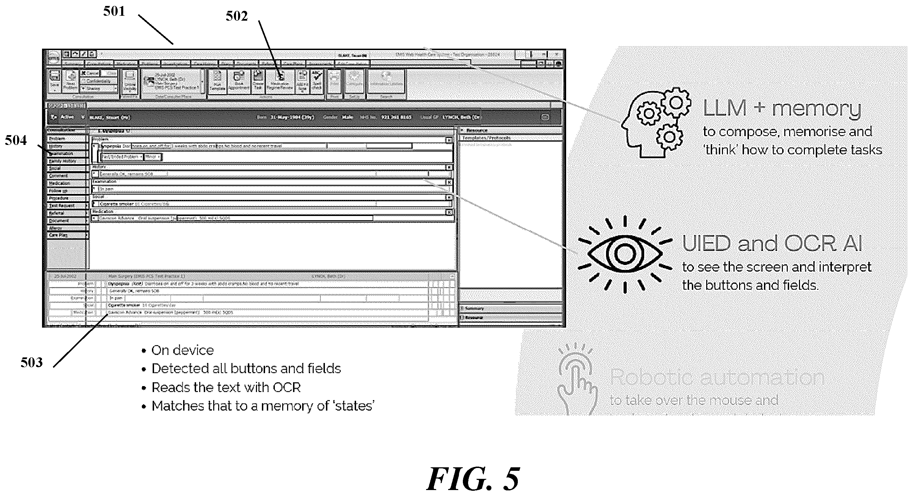

In some cases, the generating and/or executing the computer-executable program is based on determining a state of the computer. In some cases, the determining the state comprises matching the digital information to a reference in a set of reference digital information. In some cases, the set of reference digital information comprises a set of images of GUIs. In some cases, the set of reference digital information comprises a set of screenshots. In some cases, the matching comprises using a neural network. In some cases, the neural network comprises a convolutional network. In some cases, the neural network comprises an image classification model. **FIG. 5** shows an example of a GUI **501** and features of the GUI that can be detected, in accordance with some embodiments. Buttons **502,** fields **503,** text **504,** and/or other features of the GUI can be detected.

## ACTION LANGUAGE

An "action language" can refer to a representation of actions available to the action model. The representation can be a text representation, for example. The actions available can comprise the full set of available actions to the action model. The action language can represent what actions are to be done given a unique (state, task) tuple. When the action language string is retrieved, it can be passed to the action decoder to execute the actions. For example, a row in a tabular policy might be:

**Table 1.** Example of a row in a tabular policy. Given a user's intended action, and the active screen of a user interface, a sequence of actions can be carried out to perform the user's intended action.

| Intent | Screen | Actions |
|---|---|---|
| Search for pictures of Ligers | Home desktop screen | k cmd_space, t "google", k enter |

The action language in this example can be parsed to instruct the action decoder to key press the command key and hold it while pressing the space bar, which on a Mac computer opens up the finder, then type "google" in the finder bar, and finally key press enter.

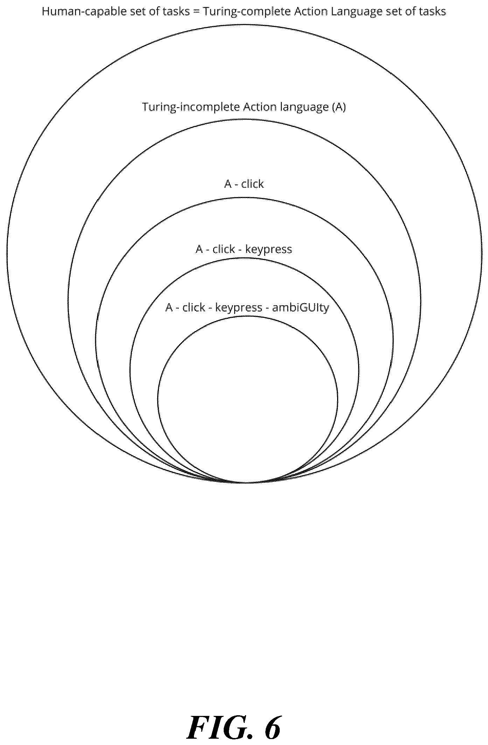

An action language can be of various levels of "completeness." **FIG. 6** illustrates the concept of Turing-completeness with respect to an action language. Completeness can refer to the fraction of computer tasks that are possible to program a tabular policy action model to do, given an action language, compared to the universe of tasks that a human would be able to perform on a computer.

As illustrated in **FIG. 6,** the completeness of an action language is reduced as features of a language are removed (the negative signs are used to indicate a "subtraction" of a feature from the language). For example, as one removes clicking from the language, then the total set of computer-controlling tasks that is programmable in that language is smaller than the set programmable by the language with clicking, since there are computer tasks that are only possible to complete with the use of the mouse click.

Designing and implementing an Action Language to be near Turing-complete is non-trivial, when one considers all the possible control flows that are possible in the language, as well as management of perceptual input, memory, and information processing. One example of a computer task that may require a high degree of Turing-completeness of the Action Language is the following: imagine a task where a customer support agent is tasked with going through their inbox every morning to handle user tickets. For each ticket, the customer support agent would classify it as either a complaint or a request. If the ticket is a complaint, the agent would write a personalized apology and solution email and send it to the user. If it is a request-type ticket, then the agent would forward the ticket to the engineering team.

To automate this task, the action language may comprise features that allow the artificial customer support agent to: Perceive the tickets on the screen and convert them to text; Use an LLM to classify the tickets as either a request or a complaint; Perform looping since repetitive tasks would be performed for multiple tickets, and the number of tickets each day is variable and unknown *a priori;* and perform conditionals since the action to take for each ticket depends on whether it is a request or a complaint.

An action language can comprise a text representation of computer control actions. Within the tabular policy, an action language can define the action sequence to be executed for each state and task objective. For example, an action sequence can be:
"t $soap, s 1, k enter, s 3, k enter, s 2, k enter, s 5"

The comma suffixed with a single space ', ' can be used as a separator between primitive actions. Other delimiters can be used as a separator, e.g., semicolons, pipes, etc. Each primitive action can comprise an action type ("enter"), and/or action arguments ("k"). The action language can have various types of actions, including, typing, clicking, sleeping, key-pressing, combination key-pressing, LLM call, looping, optical character recognition (OCR), "done" (indicating that action or task is complete), or any combination thereof.

In some cases, the action comprises typing. In some cases, the action comprises typing a character, a word, a phrase, a sentence, or a paragraph. In some cases, the action comprises a key press. In some cases, the action comprises a combination key press. In some cases, the action comprises moving a cursor. In some cases, the action comprises moving the cursor to a target position. In some cases, the action comprises clicking. In some cases, the action comprises clicking a target position. In some cases, the target position has a target UIE. In some cases, the action comprises waiting. In some cases, the action comprises waiting for a time period. In some cases, the action comprises a large language model call. In some cases, the action comprises optical character recognition. In some cases, the action comprises a loop. In some cases, the loop performs the action on an iterable data type. In some cases, the loop performs an iterable action. In some cases, the iterable action comprises a task. In some cases, the action comprises determining a state of the computer. In some cases, the action comprises determining that the task is complete. Various forms of action language for the actions are provided below by way of example. Click

A click syntax can be represented such as: c 'calendar' 3.

Such syntax takes two arguments, which are the inner text target (if available; "calendar"), and an index that identifies it in a table (e.g., ambiGUIty subtable).

*ambiGUIty*

In some cases, an action comprises a "click." A "click" action can involve a computer controlling agent to perform a mouse click action. The mouse click action can be performed on a target UIE. In some cases, an action comprises identifying a target UIE.

Identifying a target UIE presents a number of challenges. In some GUIs, an application window may have varying sizes, shapes, positions, and other varying properties that can depend on operating system of the computer, the resolution of the display, any resizing of the application in a previous session, etc. Moreover, two different target UIE can have different purposes and yet look similar, e.g., the "X" symbol for exiting the application, a sub-window within an application, or the "X" symbol on another application that has nothing to do with the application. Thus, the accuracy of identifying a target UIE may be improved when interface elements within the GUIs can be disambiguated from one another accurately. In some cases, UIEs and their properties can be stored in a table that can be referenced when identifying a target UIE. The properties can be calculated and stored such that they are size-invariant, shape-invariant, position-invariant, or invariant across another parameter that allows a target UIE to be identified accurately from another UIE. In some cases, the target UIE is identified among a set of UIEs based on a spatial characteristic of the target UIE in relation to other user interface elements. In some cases, the spatial characteristic comprises distance between the target UIE and the other UIEs in the set. In some cases, the distance comprises a relative normalized pixel distance between the target UIE and the other UIEs in the set. In some cases, the relative normalized pixel distance is stored in table for retrieval. **Table 2,** for example, shows a table storing properties of UIEs.

**Table 2.** "AmbiGUIty" tables. For various UI elements in a UI, and their positions in the UI can be predefined using a relative normalized distance.

| UI element type | Inner text | Relative normalized distance |
|---|---|---|
| icon | EMIS | 0.424 |
| text | NHS number | 0.123 |
| link | calendar | 0.093 |
| image |  | 0.982 |
| input | search | 0.239 |
| ... | ... | ... |

For illustrative purposes, a click action may take in two arguments. For example:
"c 'hello' 0"

Where c indicates the click action type, 'hello' is the inner text of the UI element to click on, and 0 is the unique index of an ambiGUIty table.

A problem can occur when there are multiple UI elements on the screen that contain the same target inner text. So in the example above of "c 'hello' 0", the action can be for the agent to click on the UIE on the screen that contains the text 'hello', but if there are multiple other buttons say that contain the inner text 'hello', then it is ambiguous to the agent which one it should click.

The "ambiguity" table can help the agent overcome this problem by uniquely identifying the correct UI element by its distance to neighboring UI elements. This can be implemented by calculating the relative normalized pixel distance between the target UI element and all other UI elements on the screen at policy generation time. These distances can be saved in an ambiGUIty table.

At action time, the saved ambiGUIty table can be retrieved using its unique index in the action language. The sum of squared errors (in relative normalized distance) can be calculated between the saved table and every ambiGUIty table generated per element containing the same target inner text.

Type

A type syntax can be represented such as: t 'hello' or t $transcript

Such syntax takes a single argument, which is either a string to be typed, or the key of Osler's key-value memory, when prefixed with a dollar sign.

Sleep

Sleep can tell the computer control execution loop to wait for some number of seconds, which is required to allow the UI to render the changes after actions are performed. A sleep syntax can be represented such as: s 3

Such syntax could be used to indicate that the agent should sleep for 3 seconds.

Key press

A key press can be represented such as: k ctrl or k ctrl_enter or k ctrl_shift_o

Key presses can be implemented with enough generality to cover all special keys, including ctrl, enter, shift, combination key presses, as well as the alphanumeric keys. This can be done by syncing up the action language with a CC library such as Enigo.

Function call

Various function calls can be incorporated into an action language. In some cases, a function call could be an LLM call (e.g., GPT). In some cases, a function call can be a call to produce a structured output.

A function call can be represented such as: llm f create-emis-note $transcript emis-note or llm p create-emis-note $transcript emis-note

Such syntax uses the first argument to indicate whether it is a function call or normal prompting. The second argument indicates the name of the LLM call that is used to find the row in the LLM table asset in order to retrieve further information such as the user message, system message, or JSON schema in the case of a function call. The third argument can be used as input to the LLM call. The fourth argument can be used as the key to store the output of the LLM call to a new entry is Osler's key-value memory. **Table 3** shows examples of action involving an LLM call.

**Table 3.** Example of actions involving an LLM call. Functions configured to perform a pre-defined task can be called by an action model. The table shows examples of functions, one of which is to fill out a SOAP based on a consultation transcript, and another of which is to generate a detailed medical letter based on the consultation transcript, and another of which is to generate details of orders based on the consultation transcript.

| Name | Vanilla prompting or function call | System message | User message | Schema |
|------|------|------|------|------|
| create-emis-note | f | JSON | Write the various sections of a SOAP note categorized by each unique problem in the consultation, from a transcript | \<serialised JSON schema for a function call to generate the EMIS note\> |
| create-letter | p | You are a professional expert medical admin assistant | From a transcript of a consultation, write a detailed medical letter | None |
| extract-orders | f | JSON | Get details of orders from a transcript of a consultation | \<serialised JSON schema for a function call to extract orders\> |

Loop

An action language can incorporate a loop action, an iterator (e.g., a hidden stack-type iterator) that uses subpolicy tables. A loop can be represented such as: loop orders place-orders

Such syntax uses the first argument as the key of an entry in Osler's key-value memory. Unlike in the type syntax where it was prefixed with a dollar sign, this argument is not a normal string so it does not need to use a prefix. The second argument can indicate the name of the subpolicy (which can be referred to as a subpolicy table). The iterator can be implicitly incrementing in the background hidden from the action language, such that every subsequent time the subpolicy is done, the iterator increments.

Loops can be implemented in the Action Language with the use of Subpolicy Tables. Subpolicy can have features similar to tabular policies, except the "task" column can replaced with a "subpolicy" column. Subpolicies can then be called from the main policy table, and they can be used to create loops in the control flow, by allowing the execution of the subpolicy as many times as the length of any selected iterable data type in the agent's memory.

## EXECUTION

In some cases, a method of the present disclosure comprises generating and/or executing a computer-executable program. In some cases, a system of the present disclosure can be configured to generate and/or execute a computer-executable program. In some cases, the generating the computer-executable program comprises generating computer readable instructions. In some cases, the generating the computer-executable program comprises generating human readable instructions. In some cases, the generating the computer-executable program comprises generating instructions in the computer's memory. In some cases, the generating the computer-executable program comprises generating instructions in the computer's storage. In some cases, the executing the computer-executable program comprises loading the computer-executable program from storage into memory.

## TABULAR POLICY

In some cases, a method of the present disclosure comprises determining an action. In some cases, a system of the present disclosure can be configured to determine an action. In some cases, the computer-executable program comprises instructions for performing an action. In some cases, the generating and executing the computer-executable program is based on determining the action. In some cases, the determining the action comprises matching the task and the state to the action in a set of possible actions. In some cases, the set of possible actions are tabulated. In some cases, the set of possible action are indexed by possible tasks and possible states.

Determining an action can comprise using a tabular policy. A tabular policy for computer control can be a table that comprises three or more columns. The three or more columns can comprise 1) screen state, 2) user command, and 3) actions. The table can be uniquely indexed by the screen state and user command. An example of tabular policy is shown in **Table 4.**

**Table 4.** Example tabular policy having task/state pairs and corresponding action sequences.

| Task | State | Actions |
|---|---|---|
| Do the note | Schedule screen | c 'patients' 0, s 2, t $patient_name, s 1, k enter, s 3 |
| Do the note | Calendar screen | c 'schedule' 1, s 1 |
| Do the note | Consultation screen | llm f create-emis-note $transcript emis-note, loop emis-note file-note, c 'save note as draft' 3 |
| Do the note | Review note draft | done |
| Do the letter | Consultation screen | llm p create-letter $transcript letter, c 'letter' 4 |
| Do the letter | Letter screen | t $letter, k enter, done |
| Place orders | Consultation screen | llm f extract-orders $soap-note orders, loop orders place-orders, c 'orders' |
| Place orders | Orders review screen | c 'save orders', done |

In some cases, an action in an action sequence can reference a subpolicy table. A subpolicy table can comprise an action sequence for another action. For example, "file-note" can be an action in the policy table. The "file-note" action can be an action sequence in the subpolicy table. An example of a subpolicy table is shown in **Table 5.**

**Table 5.** Example of a subpolicy table.

| Subpolicy name | Screen name | OCR | Actions |
|---|---|---|---|
| file-note | consultation screen | <> | k h, t $emis-note['history'], k enter, s 1, k enter, t $emis-note['examination'], k enter, s 1, k c, $emis-note[comments], k enter, c 'next problem' 5 |
| place-orders | consultation screen | <> | c 'new order', s 2 |
| place-orders | new order screen | <> | t $orders['name'], k enter, s 1, t $orders['unit'], k enter, s 1, t$orders['quantity'], k enter |

## VIRTUAL MACHINE

In some cases, a method or a system of the present disclosure can be implemented on a virtual machine. The virtual machine can allow a user to use a computer while the action model is running. For example, a medical professional may use a computer to analyze a patient's medical history while the action model is using a virtual machine to take notes or make multiple orders.

In some cases, a virtual machine can be on a cloud. In some cases, a virtual machine can be a cloud computer. In some cases, a virtual machine can be implemented within the computer. A virtual machine can comprise a same application as the computer. For example, a virtual machine can comprise the same electronic health record application as the computer. In some cases, the state of the digital information is determined on a virtual machine for the action model. In some cases, the computer-executable program is generated and/or executed on the virtual machine.

## EXPRESSION

In some cases, a method of the present disclosure comprises processing an expression produced by a user's expression. In some cases, a system of the present disclosure can be configured to process an expression produced by a user's expression. In some cases, the expressing is speaking. In some cases, the expression in speech. In some cases, the expressing is writing. In some cases, the expression is written. In some cases, the expressing is typing. In some cases, the expression is typed. In some cases, the expressing is verbal, non-verbal, or both. In some cases, an expression comprises a natural language expression. In some cases, an expression comprises natural language grammar, syntax, or both, e.g., in English.

In some cases, the expressing is directed to a human. In some cases, the expressing is directed to the computer. In some cases, the expressing is done by a medical professional. In some cases, the medical professional is a doctor, a nurse, a therapist, or an EMT. In some cases, the expressing is done by a subject. In some cases, the subject is a patient. In some cases, the subject is paralyzed. In some cases, the subject is blind.

## COMMUNICATION TO A USER

In some cases, a method of the present disclosure comprises handling an error or an exception. In some cases, a system of the present disclosure can be configured to handle an error or an exception. In some cases, a method of the present disclosure comprises communicating a result to a user. In some cases, a system of the present disclosure can be configured to communicate a result to a user. In some cases, a message can be provided to a user when the task cannot be determined or performed. In some cases, a message can be provided to a user when the expression cannot be understood. In some cases, a message can be provided to a user when the action cannot be determined or performed. In some cases, a message can be provided when the task is complete. In some cases, the message is displayed on a screen by the computer to the user. In some cases, the message is played as sound by the computer to the user.

## COMPUTING SYSTEM

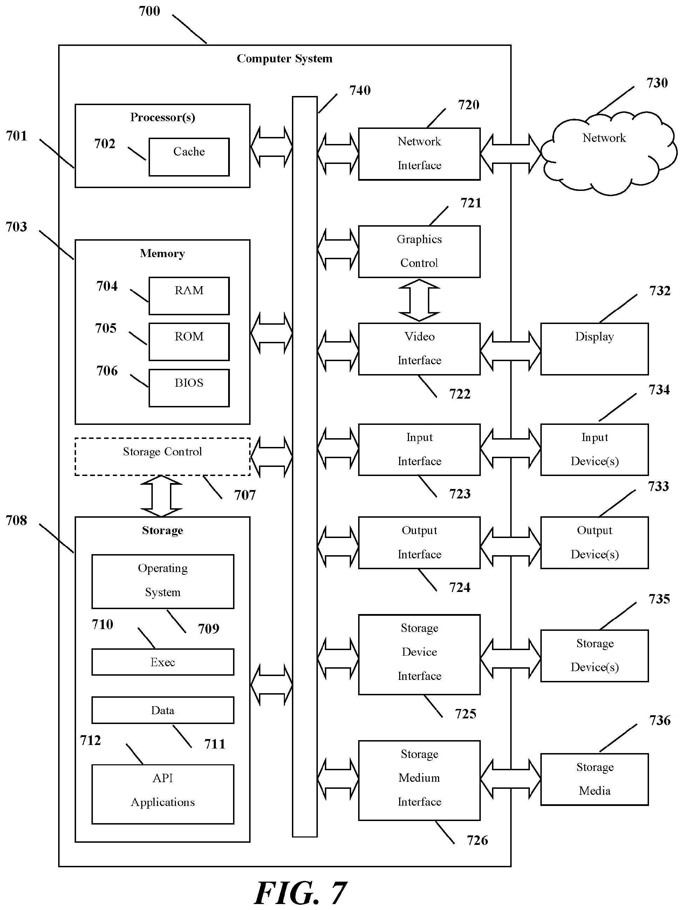

In some aspects, the present disclosure describes a computer-implemented system comprising: a digital processing device comprising: at least one processor, an operating system configured to perform executable instructions, a memory, and a computer program including instructions executable by the digital processing device to determine a task, determine a state, determine an action, and/or perform the action. In some aspects, the present disclosure describes a computer-implemented method, implementing any one of the methods disclosed herein in a computer system. Referring to **FIG. 7,** a block diagram is shown depicting an exemplary machine that includes a computer system **700** (e.g., a processing or computing system) within which a set of instructions can execute for causing a device to perform or execute any one or more of the aspects and/or methodologies for determining a task, determining a state, determining an action, and/or performing the action. The components in **FIG. 7** are examples only and do not limit the scope of use or functionality of any hardware, software, embedded logic component, or a combination of two or more such components implementing particular embodiments.

Computer system **700** may include one or more processors **701,** a memory **703,** and a storage **708** that communicate with each other, and with other components, via a bus **740.** The bus **740** may also link a display **732,** one or more input devices **733** (which may, for example, include a keypad, a keyboard, a mouse, a microphone, a stylus, etc.), one or more output devices **734,** one or more storage devices **735,** and various tangible storage media **736.** All of these elements may interface directly or via one or more interfaces or adaptors to the bus **740.** For instance, the various tangible storage media **736** can interface with the bus **740** via storage medium interface **726.** Computer system **700** may have any suitable physical form, including but not limited to one or more integrated circuits (ICs), printed circuit boards (PCBs), mobile handheld devices (such as mobile telephones or PDAs), laptop or notebook computers, distributed computer systems, computing grids, or servers.

Computer system **700** includes one or more processor(s) **701** (e.g., central processing units (CPUs), general purpose graphics processing units (GPGPUs), or quantum processing units (QPUs)) that carry out functions. Computer system **700** may be one of various high performance computing platforms. For instance, the one or more processor(s) **701** may form a high performance computing cluster. In some cases, the one or more processors **701** may form a distributed computing system connected by wired and/or wireless networks. In some cases, arrays of CPUs, GPUs, QPUs, or any combination thereof may be operably linked to implement any one of the methods disclosed herein. Processor(s) **701** optionally contains a cache memory unit **702** for temporary local storage of instructions, data, or computer addresses. Processor(s) **701** are configured to assist in execution of computer readable instructions. Computer system **700** may provide functionality for the components depicted in **FIG. 7** as a result of the processor(s) **701** executing non-transitory, processor-executable instructions embodied in one or more tangible computer-readable storage media, such as memory **703,** storage **708,** storage devices **735,** and/or storage medium **736.** The computer-readable media may store software that implements particular embodiments, and processor(s) **701** may execute the software. Memory **703** may read the software from one or more other computer-readable media (such as mass storage device(s) **735, 736)** or from one or more other sources through a suitable interface, such as network interface **720.** The software may cause processor(s) **701** to carry out one or more processes or one or more steps of one or more processes described or illustrated herein. Carrying out such processes or steps may include defining data structures stored in memory **703** and modifying the data structures as directed by the software.

The memory **703** may include various components (e.g., machine readable media) including, but not limited to, a random access memory component (e.g., RAM **704)** (e.g., static RAM (SRAM), dynamic RAM (DRAM), ferroelectric random access memory (FRAM), phase-change random access memory (PRAM), etc.), a read-only memory component (e.g., ROM **705),** and any combinations thereof. ROM **705** may act to communicate data and instructions unidirectionally to processor(s) **701,** and RAM **704** may act to communicate data and instructions bidirectionally with processor(s) **701.** ROM **705** and RAM **704** may include any suitable tangible computer-readable media described below. In one example, a basic input/output system **706** (BIOS), including basic routines that help to transfer information between elements within computer system **700,** such as during start-up, may be stored in the memory **703.**

Fixed storage **708** is connected bidirectionally to processor(s) **701,** optionally through storage control unit **707.** Fixed storage **708** provides additional data storage capacity and may also include any suitable tangible computer-readable media described herein. Storage **708** may be used to store operating system **709,** executable(s) **710,** data **711,** applications **712** (application programs), and the like. Storage **708** can also include an optical disk drive, a solid-state memory device (e.g., flash-based systems), or a combination of any of the above. Information in storage **708** may, in appropriate cases, be incorporated as virtual memory in memory **703.**

In one example, storage device(s) **735** may be removably interfaced with computer system **700** (e.g., via an external port connector (not shown)) via a storage device interface **725.** Particularly, storage device(s) **735** and an associated machine-readable medium may provide non-volatile and/or volatile storage of machine-readable instructions, data structures, program modules, and/or other data for the computer system **700.** In one example, software may reside, completely or partially, within a machine-readable medium on storage device(s) **735.** In another example, software may reside, completely or partially, within processor(s) **701.**

Bus **740** connects a wide variety of subsystems. Herein, reference to a bus may encompass one or more digital signal lines serving a common function, where appropriate. Bus **740** may be any of several types of bus structures including, but not limited to, a memory bus, a memory controller, a peripheral bus, a local bus, and any combinations thereof, using any of a variety of bus architectures. As an example, and not by way of limitation, such architectures include an Industry Standard Architecture (ISA) bus, an Enhanced ISA (EISA) bus, a Micro Channel Architecture (MCA) bus, a Video Electronics Standards Association local bus (VLB), a Peripheral Component Interconnect (PCI) bus, a PCI-Express (PCI-X) bus, an Accelerated Graphics Port (AGP) bus, HyperTransport (HTX) bus, serial advanced technology attachment (SATA) bus, and any combinations thereof.

Computer system **700** may also include an input device **733.** In one example, a user of computer system **700** may enter commands and/or other information into computer system **700** via input device(s) **733.** Examples of an input device(s) **733** include, but are not limited to, an alpha-numeric input device (e.g., a keyboard), a pointing device (e.g., a mouse or touchpad), a touchpad, a touch screen, a multi-touch screen, a joystick, a stylus, a gamepad, an audio input device (e.g., a microphone, a voice response system, etc.), an optical scanner, a video or still image capture device (e.g., a camera), and any combinations thereof. In some cases, the input device is a Kinect, Leap Motion, or the like. Input device(s) **733** may be interfaced to bus **740** via any of a variety of input interfaces **723** (e.g., input interface **723)** including, but not limited to, serial, parallel, game port, USB, FIREWIRE, THUNDERBOLT, or any combination of the above. In some cases, an input device **733** may be used to determine a task, determine a state, determine an action, and/or perform the action. In some cases, determining a task, determining a state, determining an action, and/or performing the action can comprise using human inputs through an input device **733.**

In particular embodiments, when computer system **700** is connected to network **730,** computer system **700** may communicate with other devices, specifically mobile devices and enterprise systems, distributed computing systems, cloud storage systems, cloud computing systems, and the like, connected to network **730.** Communications to and from computer system **700** may be sent through network interface **720.** For example, network interface **720** may receive incoming communications (such as requests or responses from other devices) in the form of one or more packets (such as Internet Protocol (IP) packets) from network **730,** and computer system **700** may store the incoming communications in memory **703** for processing. Computer system **700** may similarly store outgoing communications (such as requests or responses to other devices) in the form of one or more packets in memory **703** and communicated to network **730** from network interface **720.** Processor(s) **701** may access these communication packets stored in memory **703** for processing.

Examples of the network interface **720** include, but are not limited to, a network interface card, a modem, and any combination thereof. Examples of a network **730** or network segment **730** include, but are not limited to, a distributed computing system, a cloud computing system, a wide area network (WAN) (e.g., the Internet, an enterprise network), a local area network (LAN) (e.g., a network associated with an office, a building, a campus or other relatively small geographic space), a telephone network, a direct connection between two computing devices, a peer-to-peer network, and any combinations thereof. A network, such as network **730,** may employ a wired and/or a wireless mode of communication. In general, any network topology may be used.

Information and data can be displayed through a display **732.** Examples of a display **732** include, but are not limited to, a cathode ray tube (CRT), a liquid crystal display (LCD), a thin film transistor liquid crystal display (TFT-LCD), an organic liquid crystal display (OLED) such as a passive-matrix OLED (PMOLED) or active-matrix OLED (AMOLED) display, a plasma display, and any combinations thereof. The display **732** can interface to the processor(s) **701,** memory **703,** and fixed storage **708,** as well as other devices, such as input device(s) **733,** via the bus **740.** The display **732** is linked to the bus **740** via a video interface **722,** and transport of data between the display **732** and the bus **740** can be controlled via the graphics control **721.** In some cases, the display is a video projector. In some cases, the display is a head-mounted display (HMD) such as a VR headset. In further embodiments, suitable VR headsets include, by way of non-limiting examples, HTC Vive, Oculus Rift, Samsung Gear VR, Microsoft HoloLens, Razer OSVR, FOVE VR, Zeiss VR One, Avegant Glyph, Freefly VR headset, and the like. In still further embodiments, the display is a combination of devices such as those disclosed herein.

In addition to a display **732,** computer system **700** may include one or more other peripheral output devices **734** including, but not limited to, an audio speaker, a printer, a storage device, and any combinations thereof. Such peripheral output devices may be connected to the bus **740** via an output interface **724.** Examples of an output interface **724** include, but are not limited to, a serial port, a parallel connection, a USB port, a FIREWIRE port, a THUNDERBOLT port, and any combinations thereof.

In addition, or as an alternative, computer system **700** may provide functionality as a result of logic hardwired or otherwise embodied in a circuit, which may operate in place of or together with software to execute one or more processes or one or more steps of one or more processes described or illustrated herein. Reference to software in this disclosure may encompass logic, and reference to logic may encompass software. Moreover, reference to a computer-readable medium may encompass a circuit (such as an IC) storing software for execution, a circuit embodying logic for execution, or both, where appropriate. The present disclosure encompasses any suitable combination of hardware, software, or both.

Those of skill in the art will appreciate that the various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality.

The various illustrative logical blocks, modules, and circuits described in connection with the embodiments disclosed herein may be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module executed by one or more processor(s), or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such the processor can read information from, and write information to, the storage medium. In the alternative, the storage medium may be integral to the processor. The processor and the storage medium may reside in an ASIC. The ASIC may reside in a user terminal. In the alternative, the processor and the storage medium may reside as discrete components in a user terminal.

In accordance with the description herein, suitable computing devices include, by way of non-limiting examples, server computers, desktop computers, laptop computers, notebook computers, sub-notebook computers, netbook computers, netpad computers, set-top computers, media streaming devices, handheld computers, Internet appliances, mobile smartphones, and tablet computers.

In some cases, the computing device includes an operating system configured to perform executable instructions. The operating system is, for example, software, including programs and data, which manages the device's hardware and provides services for execution of applications. Those of skill in the art will recognize that suitable server operating systems include, by way of non-limiting examples, FreeBSD, OpenBSD, NetBSD®, Linux, Apple® Mac OS X Server®, Oracle® Solaris®, Windows Server®, and Novell® NetWare®. Those of skill in the art will recognize that suitable personal computer operating systems include, by way of non-limiting examples, Microsoft® Windows®, Apple® Mac OS X®, UNIX®, and UNIX-like operating systems such as GNU/Linux®. In some cases, the operating system is provided by cloud computing. Those of skill in the art will also recognize that suitable mobile smartphone operating systems include, by way of non-limiting examples, Nokia® Symbian® OS, Apple® Ios®, Research In Motion® BlackBerry OS®, Google® Android®, Microsoft® Windows Phone® OS, Microsoft® Windows Mobile® OS, Linux®, and Palm® WebOS®.

In some cases, a computer system **700** may be accessible through a user terminal to receive user commands. The user commands may include line commands, scripts, programs, etc., and various instructions executable by the computer system **700**. A computer system **700** may receive instructions to determine a task, determine a state, determine an action, and/or perform the action, or schedule a computing job for the computer system **700** to carry out any instructions.

## NON-TRANSITORY COMPUTER READABLE STORAGE MEDIUM

In some aspects, the present disclosure describes a non-transitory computer-readable storage media encoded with a computer program including instructions executable by one or more processors to determine a task, determine a state, determine an action, and/or perform the action using any one of the methods disclosed herein. In some cases, a non-transitory computer-readable storage media may comprise instructions for determining a task, determining a state, determining an action, and/or performing the action. In some cases, the platforms, systems, media, and methods disclosed herein include one or more non-transitory computer readable storage media encoded with a program including instructions executable by the operating system of an optionally networked computing device.

In further embodiments, a computer readable storage medium is a tangible component of a computing device. In still further embodiments, a computer readable storage medium is optionally removable from a computing device. In some cases, a computer readable storage medium includes, by way of non-limiting examples, flash memory devices, solid state memory, magnetic disk drives, magnetic tape drives, optical disk drives, distributed computing systems including cloud computing systems and services, and the like. In some cases, the program and instructions are permanently, substantially permanently, semi-permanently, or non-transitorily encoded on the media.

## COMPUTER PROGRAM

In some aspects, the present disclosure describes a computer program product comprising a computer-readable medium having computer-executable code encoded therein, the computer-executable code adapted to be executed to implement any one of the methods disclosed herein. In some cases, the platforms, systems, media, and methods disclosed herein include at least one computer program, or use of the same.

A computer program includes a sequence of instructions, executable by one or more processor(s) of the computing device's CPU, written to perform a specified task. Computer readable instructions may be implemented as program modules, such as functions, objects, Application Programming Interfaces (APIs), computing data structures, and the like, which perform particular tasks or implement particular abstract data types. In light of the disclosure provided herein, those of skill in the art will recognize that a computer program may be written in various versions of various languages. In some cases, APIs may comprise various languages, for example, languages in various releases of TensorFlow, Theano, Keras, PyTorch, or any combination thereof which may be implemented in various releases of Python, Python3, C, C#, C++, MatLab, R, Java, or any combination thereof.

The functionality of the computer readable instructions may be combined or distributed as desired in various environments. In some cases, a computer program comprises one sequence of instructions. In some cases, a computer program comprises a plurality of sequences of instructions. In some cases, a computer program is provided from one location. In other embodiments, a computer program is provided from a plurality of locations. In various embodiments, a computer program includes one or more software modules. In various embodiments, a computer program includes, in part or in whole, one or more web applications, one or more standalone applications, one or more web browser plug-ins, extensions, add-ins, or add-ons, or combinations thereof.

## WEB APPLICATION

In some cases, a computer program includes a web application. In some cases, a user may enter a query for determining a task, determining a state, determining an action, and/or performing the action through a web application. In some cases, a user may determine a task, determine a state, determine an action, and/or perform the action through a web application. In light of the disclosure provided herein, those of skill in the art will recognize that a web application, in various embodiments, utilizes one or more software frameworks and one or more database systems. In some cases, a web application is created upon a software framework such as Microsoft® .NET or Ruby on Rails (RoR). In some cases, a web application utilizes one or more database systems including, by way of non-limiting examples, relational, non-relational, object oriented, associative, XML, and document oriented database systems. In further embodiments, suitable relational database systems include, by way of non-limiting examples, Microsoft® SQL Server, mySQL™, and Oracle®. Those of skill in the art will also recognize that a web application, in various embodiments, is written in one or more versions of one or more languages. A web application may be written in one or more markup languages, presentation definition languages, client-side scripting languages, server-side coding languages, database query languages, or combinations thereof. In some cases, a web application is written to some extent in a markup language such as Hypertext Markup Language (HTML), Extensible Hypertext Markup Language (XHTML), or eXtensible Markup Language (XML). In some cases, a web application is written to some extent in a presentation definition language such as Cascading Style Sheets (CSS). In some cases, a web application is written to some extent in a client-side scripting language such as Asynchronous JavaScript and XML (AJAX), Flash® ActionScript, JavaScript, or Silverlight®. In some cases, a web application is written to some extent in a server-side coding language such as Active Server Pages (ASP), ColdFusion®, Perl, Java™, JavaServer Pages (JSP), Hypertext Preprocessor (PHP), Python™, Ruby, Tcl, Smalltalk, WebDNA®, or Groovy. In some cases, a web application is written to some extent in a database query language such as Structured Query Language (SQL). In some cases, a web application integrates enterprise server products such as IBM® Lotus Domino®.

## MOBILE APPLICATION

In some cases, a computer program includes a mobile application provided to a mobile computing device. In some cases, the mobile application is provided to a mobile computing device at the time it is manufactured. In other embodiments, the mobile application is provided to a mobile computing device via the computer network described herein.

In view of the disclosure provided herein, a mobile application is created by techniques known to those of skill in the art using hardware, languages, and development environments known to the art. Those of skill in the art will recognize that mobile applications are written in several languages. Suitable programming languages include, by way of non-limiting examples, C, C++, C#, Objective-C, Java™, JavaScript, Pascal, Object Pascal, Python™, Ruby, VB.NET, WML, and XHTML/HTML with or without CSS, or combinations thereof.

Suitable mobile application development environments are available from several sources. Commercially available development environments include, by way of non-limiting examples, AirplaySDK, alcheMo, Appcelerator®, Celsius, Bedrock, Flash Lite, .NET Compact Framework, Rhomobile, and WorkLight Mobile Platform. Other development environments are available without cost including, by way of non-limiting examples, Lazarus, MobiFlex, MoSync, and PhoneGap. Also, mobile device manufacturers distribute software developer kits including, by way of non-limiting examples, iPhone and iPad (Ios) SDK, Android™ SDK, BlackBerry® SDK, BREW SDK, Palm® OS SDK, Symbian SDK, webOS SDK, and Windows® Mobile SDK.

## STANDALONE APPLICATION

In some cases, a computer program includes a standalone application, which is a program that is run as an independent computer process, not an add-on to an existing process, e.g., not a plug-in. Those of skill in the art will recognize that standalone applications are often compiled. A compiler is a computer program(s) that transforms source code written in a programming language into binary object code such as assembly language or machine code. Suitable compiled programming languages include, by way of non-limiting examples, C, C++, Objective-C, COBOL, Delphi, Eiffel, Java™, Lisp, Python™, Visual Basic, and VB .NET, or combinations thereof. Compilation is often performed, at least in part, to create an executable program. In some cases, a computer program includes one or more executable complied applications.

## SOFTWARE MODULES

In some cases, the platforms, systems, media, and methods disclosed herein include software, server, and/or database modules, or use of the same. In view of the disclosure provided herein, software modules are created by techniques known to those of skill in the art using machines, software, and languages known to the art. The software modules disclosed herein are implemented in a multitude of ways. In various embodiments, a software module comprises a file, a section of code, a programming object, a programming structure, a distributed computing resource, a cloud computing resource, or combinations thereof. In further various embodiments, a software module comprises a plurality of files, a plurality of sections of code, a plurality of programming objects, a plurality of programming structures, a plurality of distributed computing resources, a plurality of cloud computing resources, or combinations thereof. In various embodiments, the one or more software modules comprise, by way of non-limiting examples, a web application, a mobile application, a standalone application, and a distributed or cloud computing application. In some cases, software modules are in one computer program or application. In other embodiments, software modules are in more than one computer program or application. In some cases, software modules are hosted on one machine. In other embodiments, software modules are hosted on more than one machine. In further embodiments, software modules are hosted on a distributed computing platform such as a cloud computing platform. In some cases, software modules are hosted on one or more machines in one location. In other embodiments, software modules are hosted on one or more machines in more than one location.

## DATABASES

In some cases, the platforms, systems, media, and methods disclosed herein include one or more databases, or use of the same. In view of the disclosure provided herein, those of skill in the art will recognize that many databases are suitable for storage and retrieval of information about determining a task, determining a state, determining an action, and/or performing the action, or any combination thereof. In various embodiments, suitable databases include, by way of non-limiting examples, relational databases, non-relational databases, object oriented databases, object databases, entity-relationship model databases, associative databases, XML databases, document oriented databases, and graph databases. Further non-limiting examples include SQL, PostgreSQL, MySQL, Oracle, DB2, Sybase, and MongoDB. In some cases, a database is Internet-based. In further embodiments, a database is web-based. In still further embodiments, a database is cloud computing-based. In a particular embodiment, a database is a distributed database. In other embodiments, a database is based on one or more local computer storage devices.

## LIST OF EMBODIMENTS

The following list of embodiments of the invention are to be considered as disclosing various features of the invention, which features can be considered to be specific to the particular embodiment under which they are discussed, or which are combinable with the various other features as listed in other embodiments. Thus, simply because a feature is discussed under one particular embodiment does not necessarily limit the use of that feature to that embodiment.

Embodiment 1. A method of performing a task by expressing to a computer configured to perform the task by generating and executing a computer-executable program that is based on (a) determining the task from the expressing and (b) obtaining digital information displayed by the computer.

Embodiment 2. A computer-implemented method of performing a task based on a user expression, comprising generating and executing a computer-executable program based on (a) determining the task from the expression and (b) obtaining digital information displayed by a computer.

Embodiment 3. The method of Embodiment 1 or 2, wherein the determining the task comprises receiving an expression produced by the expressing through a receiver.

Embodiment 4. The method of any one of Embodiments 1-3, wherein the determining the task comprises processing the expression using a neural network.

Embodiment 5. The method of Embodiment 4, wherein the neural network comprises a large language model.

Embodiment 6. The method of Embodiment 4 or 5, wherein the neural network comprises a natural language model.

Embodiment 7. The method of any one of Embodiments 1-6, wherein the determining the task comprises generating a transcript of an expression produced by the expressing.

Embodiment 8. The method of Embodiment 7, wherein the determining the task comprises generating a prediction of the task based on the expression or the transcript.

Embodiment 9. The method of any one of Embodiments 1-8, wherein the obtaining the digital information comprises obtaining an image of a graphical user interface (GUI).

Embodiment 10. The method of Embodiment 9, wherein the image is shown on a display in electronic communication with the computer.

Embodiment 11. The method of Embodiment 9 or 10, wherein the image comprises a screenshot of the display.

Embodiment 12. The method of any one of Embodiments 9-11, wherein the image comprises pixels.

Embodiment 13. The method of any one of Embodiments 9-12, wherein the image comprises a vector graphic.

Embodiment 14. The method of any one of Embodiments 9-13, wherein the image is a digital representation of information shown on the display.

Embodiment 15. The method of any one of Embodiments 9-14, wherein the obtaining the digital information comprises detecting a user interface element (UIE) in the image.

Embodiment 16. The method of any one of Embodiments 9-15, wherein the obtaining the digital information comprises generating a sequence representation of the image.

Embodiment 17. The method of Embodiment 16, wherein the sequence representation comprises text.

Embodiment 18. The method of Embodiment 16 or 17, wherein the sequence representation comprises bits.

Embodiment 19. The method of any one of Embodiments 16-18, wherein the sequence representation comprises a property of the UIE.

Embodiment 20. The method of Embodiment 19, wherein the property comprise a class of the UIE, a bounding box of the UIE, a position of the UIE, a text in the UIE, or any combination thereof.

Embodiment 21. The method of any one of Embodiments 1-20, wherein the generating and executing the computer-executable program is based on determining a state of the computer.

Embodiment 22. The method of Embodiment 21, wherein the determining the state comprises matching the digital information to a reference in a set of reference digital information.

Embodiment 23. The method of Embodiment 22, wherein the set of reference digital information comprises a set of images of GUIs.

Embodiment 24. The method of Embodiment 22 or 23, wherein the set of reference digital information comprises a set of screenshots.

Embodiment 25. The method of any one of Embodiments 22-24, wherein the matching comprises using a neural network.

Embodiment 26. The method of Embodiment 25, wherein the neural network comprises a convolutional network.

Embodiment 27. The method of any one of Embodiments 1-26, wherein the computer-executable program comprises instructions for performing an action.

Embodiment 28. The method of Embodiment 27, wherein the generating and executing the computer-executable program is based on determining the action.

Embodiment 29. The method of Embodiment 28, wherein the determining the action comprises matching the task and the state to the action in a set of possible actions.

Embodiment 30. The method of Embodiment 29, wherein the set of possible actions are tabulated.

Embodiment 31. The method of Embodiment 29 or 30, wherein the set of possible action are indexed by possible tasks and possible states.

Embodiment 32. The method of any one of Embodiments 29-31, wherein the action identifies a target UIE.

Embodiment 33. The method of Embodiment 32, wherein the target UIE is identified among a set of UIEs based on a spatial characteristic of the target UIE in relation to other user interface elements.

Embodiment 34. The method of Embodiment 33, wherein the spatial characteristic comprises distance between the target UIE and the other UIEs in the set.

Embodiment 35. The method of Embodiment 34, wherein the distance comprises a relative normalized pixel distance between the target UIE and the other UIEs in the set.

Embodiment 36. The method of Embodiment 35, wherein the relative normalized pixel distance is stored in table for retrieval.

Embodiment 37. The method of any one of Embodiments 27-36, wherein the action comprises typing.

Embodiment 38. The method of Embodiment 37, wherein the action comprises typing a character, a word, a phrase, a sentence, or a paragraph.

Embodiment 39. The method of any one of Embodiments 27-38, wherein the action comprises a key press.

Embodiment 40. The method of Embodiment 39, wherein the action comprises a combination key press.

Embodiment 41. The method of any one of Embodiments 27-40, wherein the action comprises moving a cursor.

Embodiment 42. The method of Embodiment 41, wherein the action comprises moving the cursor to a target position.

Embodiment 43. The method of any one of Embodiments 27-42, wherein the action comprises clicking.

Embodiment 44. The method of Embodiment 43, wherein the action comprises clicking a target position.

Embodiment 45. The method of Embodiment 43 or 44, wherein the target position has a target UIE.

Embodiment 46. The method of any one of Embodiments 27-45, wherein the action comprises waiting.

Embodiment 47. The method of Embodiment 46, wherein the action comprises waiting for a time period.

Embodiment 48. The method of any one of Embodiments 27-47, wherein the action comprises a large language model call.

Embodiment 49. The method of any one of Embodiments 27-48, wherein the action comprises optical character recognition.

Embodiment 50. The method of any one of Embodiments 27-49, wherein the action comprises determining a state of the computer.

Embodiment 51. The method of any one of Embodiments 27-50, wherein the action comprises determining that the task is complete.

Embodiment 52. The method of any one of Embodiments 27-51, wherein the action comprises a loop.

Embodiment 53. The method of Embodiment 52, wherein the loop performs the action on an iterable data type.

Embodiment 54. The method of Embodiment 52 or 53, wherein the loop performs an iterable action, an iterable task, or both.

Embodiment 55. The method of any one of Embodiments 1-54, wherein the state of the digital information is determined on a virtual machine.

Embodiment 56. The method of any one of Embodiments 1-55, wherein the computer-executable program is executed on a virtual machine.

Embodiment 57. The method of any one of Embodiments 1-56, wherein the task comprises dictating.

Embodiment 58. The method of Embodiment 57, wherein the task comprises transcribing.

Embodiment 59. The method of Embodiment 57 or 58, wherein the dictating comprises dictating not every spoken word.

Embodiment 60. The method of any one of Embodiments 57-59, wherein the dictating comprises dictating a subset of the spoken words.

Embodiment 61. The method of any one of Embodiments 57-60, wherein the dictating comprises dictating while improving grammar.

Embodiment 62. The method of any one of Embodiments 1-61, wherein the task comprises searching.

Embodiment 63. The method of Embodiment 62, wherein the task comprises searching a database.

Embodiment 64. The method of Embodiment 62 or 63, wherein the task comprises searching a search engine.

Embodiment 65. The method of any one of Embodiments 1-64, wherein the task comprises opening a document.

Embodiment 66. The method of any one of Embodiments 1-65, wherein the task comprises ordering an item.

Embodiment 67. The method of Embodiment 66, wherein the item is a medical item.

Embodiment 68. The method of Embodiment 66 or 67, wherein the item is a prescription, an X-ray, a test, a discharge instruction, an examination, or any combination thereof.

Embodiment 69. The method of any one of Embodiments 1-68, wherein the task comprises taking notes.

Embodiment 70. The method of any one of Embodiments 1-69, wherein the task comprises writing an email or a text message.

Embodiment 71. The method of any one of Embodiments 1-70, wherein the task comprises sending a voice recording.

Embodiment 72. The method of any one of Embodiments 1-71, wherein the task comprises calling for a medical professional.

Embodiment 73. The method of any one of Embodiments 1-72, wherein the task comprises interacting with medical software.

Embodiment 74. The method of any one of Embodiments 1-73, wherein the expressing is speaking.

Embodiment 75. The method of any one of Embodiments 1-74, wherein an expression produced by the expressing is speech.

Embodiment 76. The method of any one of Embodiments 1-75, wherein the expressing is writing.

Embodiment 77. The method of any one of Embodiments 1-76, wherein an expression produced by the expressing is written.

Embodiment 78. The method of any one of Embodiments 1-77, wherein the expressing is typing.

Embodiment 79. The method of any one of Embodiments 1-78, wherein an expression produced by the expressing is typed.

Embodiment 80. The method of any one of Embodiments 1-79, wherein the expressing is verbal, non-verbal, or both.

Embodiment 81. The method of any one of Embodiments 1-80, wherein the expressing is directed to a human.

Embodiment 82. The method of any one of Embodiments 1-81, wherein the expressing is directed to the computer.

Embodiment 83. The method of any one of Embodiments 1-82, wherein the expressing is done by a medical professional.

Embodiment 84. The method of Embodiment 83, wherein the medical professional is a doctor, a nurse, a therapist, or an EMT.

Embodiment 85. The method of any one of Embodiments 1-84, wherein the expressing is done by a subject.

Embodiment 86. The method of Embodiment 85, wherein the subject is a patient.

Embodiment 87. The method of Embodiment 85 or 86, wherein the subject is paralyzed.

Embodiment 88. The method of any one of Embodiments 85-87, wherein the subject is blind.

Embodiment 89. The method of any one of Embodiments 1-88, wherein the computer comprises a smartphone, a tablet, a laptop, a desktop, a server, or a cloud.

Embodiment 90. The method of any one of Embodiments 1-89, wherein the computer comprises a mouse, a keyboard, and a display.

Embodiment 91. The method of any one of Embodiments 3-90, wherein the receiver comprises a mic.

Embodiment 92. The method of any one of Embodiments 3-91, wherein the receiver comprises a keyboard.

Embodiment 93. The method of any one of Embodiments 3-92, wherein the receiver comprises a camera.

Embodiment 94. The method of any one of Embodiments 1-93, wherein the generating the computer-executable program comprises generating computer readable instructions.

Embodiment 95. The method of any one of Embodiments 1-94, wherein the generating the computer-executable program comprises generating human readable instructions.

Embodiment 96. The method of any one of Embodiments 1-95, wherein the generating the computer-executable program comprises generating instructions in the computer's memory.

Embodiment 97. The method of any one of Embodiments 1-96, wherein the generating the computer-executable program comprises generating instructions in the computer's storage.

Embodiment 98. The method of any one of Embodiments 1-97, wherein the executing the computer-executable program comprises loading the computer-executable program from storage into memory.

Embodiment 99. The method of any one of Embodiments 1-98, wherein the method further comprises providing a message to a user when the task cannot be determined or performed.

Embodiment 100. The method of any one of Embodiments 1-99, wherein the method further comprises providing a message to a user when the expression cannot be understood.

Embodiment 101. The method of any one of Embodiments 1-100, wherein the method further comprises providing a message to a user when the action cannot be determined or performed.

Embodiment 102. The method of any one of Embodiments 1-101, wherein the method further comprises providing a message when the task is complete.

Embodiment 103. The method of any one of Embodiments 1-102, wherein the message is displayed on a screen by the computer to the user.

Embodiment 104. The method of any one of Embodiments 1-103, wherein the message is played as sound by the computer to the user.

Embodiment 105. A computer-implemented system comprising: a digital processing device comprising: at least one processor, an operating system configured to perform executable instructions, a memory, and a computer program including instructions executable by the digital processing device to perform a computer task by a user expressing to the computer-implemented system, and wherein the computer-implemented system is configured to perform the task by generating and executing a computer-executable program, comprising:

(a) determining the task from the expressing; and

(b) obtaining digital information displayed by the computer.

Embodiment 106. A computer-implemented system comprising: a digital processing device comprising: at least one processor, an operating system configured to perform executable instructions, a memory, and a computer program including instructions executable by the digital processing device to perform a computer task based on a user expression, and wherein the computer-implemented system is configured to perform the task by generating and executing a computer-executable program, comprising:

(a) determining the task from the expression; and

(b) obtaining digital information displayed by the computer.

Embodiment 107. The system of Embodiment 105 or 106, wherein the determining the task comprises receiving an expression produced by the expressing through a receiver.

Embodiment 108. The system of any one of Embodiments 105-107, wherein the determining the task comprises processing the expression using a neural network.

Embodiment 109. The system of Embodiment 108, wherein the neural network comprises a large language model.

Embodiment 110. The system of Embodiment 108 or 109, wherein the neural network comprises a natural language model.

Embodiment 111. The system of any one of Embodiments 105-110, wherein the determining the task comprises generating a transcript of an expression produced by the expressing.

Embodiment 112. The system of Embodiment 111, wherein the determining the task comprises generating a prediction of the task based on the expression or the transcript.

Embodiment 113. The system of any one of Embodiments 105-112, wherein the obtaining the digital information comprises obtaining an image of a graphical user interface (GUI).

Embodiment 114. The system of Embodiment 113, wherein the image is shown on a display in electronic communication with the computer.

Embodiment 115. The system of Embodiment 113 or 114, wherein the image comprises a screenshot of the display.

Embodiment 116. The system of any one of Embodiments 113-115, wherein the image comprises pixels.

Embodiment 117. The system of any one of Embodiments 113-116, wherein the image comprises a vector graphic.

Embodiment 118. The system of any one of Embodiments 113-117, wherein the image is a digital representation of information shown on the display.

Embodiment 119. The system of any one of Embodiments 113-118, wherein the obtaining the digital information comprises detecting a user interface element (UIE) in the image.

Embodiment 120. The system of any one of Embodiments 113-119, wherein the obtaining the digital information comprises generating a sequence representation of the image.

Embodiment 121. The system of Embodiment 120, wherein the sequence representation comprises text.

Embodiment 122. The system of Embodiment 120 or 121, wherein the sequence representation comprises bits.

Embodiment 123. The system of any one of Embodiments 120-122, wherein the sequence representation comprises a property of the UIE.

Embodiment 124. The system of Embodiment 123, wherein the property comprise a class of the UIE, a bounding box of the UIE, a position of the UIE, a text in the UIE, or any combination thereof.

Embodiment 125. The system of any one of Embodiments 105-124, wherein the generating and executing the computer-executable program is based on determining a state of the computer.

Embodiment 126. The system of Embodiment 125, wherein the determining the state comprises matching the digital information to a reference in a set of reference digital information.

Embodiment 127. The system of Embodiment 126, wherein the set of reference digital information comprises a set of images of GUIs.

Embodiment 128. The system of Embodiment 126 or 127, wherein the set of reference digital information comprises a set of screenshots.

Embodiment 129. The system of any one of Embodiments 126-128, wherein the matching comprises using a neural network.

Embodiment 130. The system of Embodiment 129, wherein the neural network comprises a convolutional network.

Embodiment 131. The system of any one of Embodiments 105-130, wherein the computer-executable program comprises instructions for performing an action.

Embodiment 132. The system of Embodiment 131, wherein the generating and executing the computer-executable program is based on determining the action.

Embodiment 133. The system of Embodiment 132, wherein the determining the action comprises matching the task and the state to the action in a set of possible actions.

Embodiment 134. The system of Embodiment 133, wherein the set of possible actions are tabulated.

Embodiment 135. The system of Embodiment 133 or 134, wherein the set of possible action are indexed by possible tasks and possible states.

Embodiment 136. The system of any one of Embodiments 133-135, wherein the action identifies a target UIE.

Embodiment 137. The system of Embodiment 136, wherein the target UIE is identified among a set of UIEs based on a spatial characteristic of the target UIE in relation to other user interface elements.

Embodiment 138. The system of Embodiment 137, wherein the spatial characteristic comprises distance between the target UIE and the other UIEs in the set.

Embodiment 139. The system of Embodiment 138, wherein the distance comprises a relative normalized pixel distance between the target UIE and the other UIEs in the set.

Embodiment 140. The system of Embodiment 139, wherein the relative normalized pixel distance is stored in table for retrieval.

Embodiment 141. The system of any one of Embodiments 131-140, wherein the action comprises typing.

Embodiment 142. The system of Embodiment 141, wherein the action comprises typing a character, a word, a phrase, a sentence, or a paragraph.

Embodiment 143. The system of any one of Embodiments 131-142, wherein the action comprises a key press.

Embodiment 144. The system of Embodiment 143, wherein the action comprises a combination key press.

Embodiment 145. The system of any one of Embodiments 131-144, wherein the action comprises moving a cursor.

Embodiment 146. The system of Embodiment 145, wherein the action comprises moving the cursor to a target position.

Embodiment 147. The system of any one of Embodiments 131-146, wherein the action comprises clicking.

Embodiment 148. The system of Embodiment 147, wherein the action comprises clicking a target position.

Embodiment 149. The system of Embodiment 147 or 148, wherein the target position has a target UIE.

Embodiment 150. The system of any one of Embodiments 131-149, wherein the action comprises waiting.

Embodiment 151. The system of Embodiment 150, wherein the action comprises waiting for a time period.

Embodiment 152. The system of any one of Embodiments 131-151, wherein the action comprises a large language model call.

Embodiment 153. The system of any one of Embodiments 131-152, wherein the action comprises optical character recognition.

Embodiment 154. The system of any one of Embodiments 131-153, wherein the action comprises determining a state of the computer.

Embodiment 155. The system of any one of Embodiments 131-154, wherein the action comprises determining that the task is complete.

Embodiment 156. The system of any one of Embodiments 131-155, wherein the action comprises a loop.

Embodiment 157. The system of Embodiment 156, wherein the loop performs the action on an iterable data type.

Embodiment 158. The system of Embodiment 156 or 157, wherein the loop performs an iterable action, an iterable task, or both.

Embodiment 159. The system of any one of Embodiments 105-158, wherein the state of the digital information is determined on a virtual machine.

Embodiment 160. The system of any one of Embodiments 105-159, wherein the computer-executable program is executed on a virtual machine.

Embodiment 161. The system of any one of Embodiments 105-160, wherein the task comprises dictating.

Embodiment 162. The system of Embodiment 161, wherein the task comprises transcribing.

Embodiment 163. The system of Embodiment 161 or 162, wherein the dictating comprises dictating not every spoken word.

Embodiment 164. The system of any one of Embodiments 161-163, wherein the dictating comprises dictating a subset of the spoken words.

Embodiment 165. The system of any one of Embodiments 161-164, wherein the dictating comprises dictating while improving grammar.

Embodiment 166. The system of any one of Embodiments 105-165, wherein the task comprises searching.

Embodiment 167. The system of Embodiment 166, wherein the task comprises searching a database.

Embodiment 168. The system of Embodiment 166 or 167, wherein the task comprises searching a search engine.

Embodiment 169. The system of any one of Embodiments 105-168, wherein the task comprises opening a document.

Embodiment 170. The system of any one of Embodiments 105-169, wherein the task comprises ordering an item.

Embodiment 171. The system of Embodiment 170, wherein the item is a medical item.

Embodiment 172. The system of Embodiment 170 or 171, wherein the item is a prescription, an X-ray, a test, a discharge instruction, an examination, or any combination thereof.

Embodiment 173. The system of any one of Embodiments 105-172, wherein the task comprises taking notes.

Embodiment 174. The system of any one of Embodiments 105-173, wherein the task comprises writing an email or a text message.

Embodiment 175. The system of any one of Embodiments 105-174, wherein the task comprises sending a voice recording.

Embodiment 176. The system of any one of Embodiments 105-175, wherein the task comprises calling for a medical professional.

Embodiment 177. The system of any one of Embodiments 105-176, wherein the task comprises interacting with medical software.

Embodiment 178. The system of any one of Embodiments 105-177, wherein the expressing is speaking.

Embodiment 179. The system of any one of Embodiments 105-178, wherein an expression produced by the expressing is speech.

Embodiment 180. The system of any one of Embodiments 105-179, wherein the expressing is writing.

Embodiment 181. The system of any one of Embodiments 105-180, wherein an expression produced by the expressing is written.

Embodiment 182. The system of any one of Embodiments 105-181, wherein the expressing is typing.

Embodiment 183. The system of any one of Embodiments 105-182, wherein an expression produced by the expressing is typed.

Embodiment 184. The system of any one of Embodiments 105-183, wherein the expressing is verbal, non-verbal, or both.

Embodiment 185. The system of any one of Embodiments 105-184, wherein the expressing is directed to a human.

Embodiment 186. The system of any one of Embodiments 105-185, wherein the expressing is directed to the computer.

Embodiment 187. The system of any one of Embodiments 105-186, wherein the expressing is done by a medical professional.

Embodiment 188. The system of Embodiment 187, wherein the medical professional is a doctor, a nurse, a therapist, or an EMT.

Embodiment 189. The system of any one of Embodiments 105-188, wherein the expressing is done by a subject.

Embodiment 190. The system of Embodiment 189, wherein the subject is a patient.

Embodiment 191. The system of Embodiment 189 or 190, wherein the subject is paralyzed.

Embodiment 192. The system of any one of Embodiments 189-191, wherein the subject is blind.

Embodiment 193. The system of any one of Embodiments 105-193, wherein the computer comprises a smartphone, a tablet, a laptop, a desktop, a server, or a cloud.

Embodiment 194. The system of any one of Embodiments 105-193, wherein the computer comprises a mouse, a keyboard, and a display.

Embodiment 195. The system of any one of Embodiments 107-194, wherein the receiver comprises a mic.

Embodiment 196. The system of any one of Embodiments 107-195, wherein the receiver comprises a keyboard.

Embodiment 197. The system of any one of Embodiments 107-196, wherein the receiver comprises a camera.

Embodiment 198. The system of any one of Embodiments 105-197, wherein the generating the computer-executable program comprises generating computer readable instructions.

Embodiment 199. The system of any one of Embodiments 105-198, wherein the generating the computer-executable program comprises generating human readable instructions.

Embodiment 200. The system of any one of Embodiments 105-199, wherein the generating the computer-executable program comprises generating instructions in the computer's memory.

Embodiment 201. The system of any one of Embodiments 105-200, wherein the generating the computer-executable program comprises generating instructions in the computer's storage.

Embodiment 202. The system of any one of Embodiments 105-201, wherein the executing the computer-executable program comprises loading the computer-executable program from storage into memory.

Embodiment 203. The system of any one of Embodiments 105-202, wherein the computer program including the instructions further comprises providing a message to a user when the task cannot be determined or performed.

Embodiment 204. The system of any one of Embodiments 105-203, wherein the computer program including the instructions further comprises providing a message to a user when the expression cannot be understood.

Embodiment 205. The system of any one of Embodiments 105-204, wherein the computer program including the instructions further comprises providing a message to a user when the action cannot be determined or performed.

Embodiment 206. The system of any one of Embodiments 105-205, wherein the computer program including the instructions further comprises providing a message when the task is complete.

Embodiment 207. The system of any one of Embodiments 105-206, wherein the message is displayed on a screen by the computer to the user.

Embodiment 208. The system of any one of Embodiments 105-207, wherein the message is played as sound by the computer to the user.

Embodiment 209. The system of any one of Embodiments 105-208, further comprising a display.

Embodiment 210. The system of any one of Embodiments 105-209, further comprising a receiver.

Embodiment 211. The system of any one of Embodiments 105-210, further comprising a storage.

Embodiment 212. The system of any one of Embodiments 105-211, wherein the system is a cloud-computing system.

Embodiment 213. The system of any one of Embodiments 105-212, wherein the system comprises a virtual machine.

Embodiment 214. A method of performing a click by expressing to a computer configured to perform the click by generating and executing a computer-executable program that is based on distinguishing the correct click target that comprises the same text as an incorrect click target.

Embodiment 215. A computer-implemented method of performing a click based on a user expression, comprising generating and executing a computer-executable program based on distinguishing the correct click target that comprises the same text as an incorrect click target.

Embodiment 216. A computer-implemented system comprising: a digital processing device comprising: at least one processor, an operating system configured to perform executable instructions, a memory, and a computer program including instructions executable by the digital processing device to perform a click by a user expressing to the computer-implemented system, and wherein the computer-implemented system is configured to perform the click by generating and executing a computer-executable program based on distinguishing the correct click target that comprises the same text as an incorrect click target.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the present disclosure may be employed in practicing the present disclosure. It is intended that the following claims define the scope of the present disclosure and that methods and structures within the scope of these claims and their equivalents be covered thereby.

**Claims**

1. A computer-implemented system comprising: a digital processing device comprising: at least one processor, an operating system configured to perform executable instructions, a memory, and a computer program including instructions executable by the digital processing device to perform a computer task by a user expressing an expression to the computer-implemented system, and wherein the computer-implemented system is configured to perform the task by generating and executing a computer-executable program, comprising:

   (a) determining the computer task from the expression, by:

      i. receiving the user's expression through a receiver in electronic communication with the computer-implemented system; and
      ii. processing the user's expression using a large language model to generate a prediction of the computer task that is desired by the user based on the user's expression, wherein the computer task involves performing an action using an electronic medical record application, and wherein the prediction is non-deterministic;

   (b) determining a state of digital information displayed on a display in electronic communication with the computer-implemented system;
   (c) determining the action to be performed, by matching the computer task and the state to the action in a set of enumerated actions, wherein the set of enumerated actions are indexed by computer tasks and display states, and wherein the matching is deterministic; and
   (d) generating and executing a computer-executable program that comprises instructions for performing the action, thereby performing the computer task.

2. The computer-implemented system of claim 1, wherein the determining the state of the digital information comprises obtaining an image of a graphical user interface (GUI), optionally wherein the image comprises pixels and/or optionally wherein the image is shown on a display in electronic communication with the computer, further optionally wherein the image is a digital representation of information shown on the display.

3. The computer-implemented system of claim 1 or 2, wherein the determining the state of the digital information comprises detecting a user interface element (UIE) in the image.

4. The computer-implemented system of claim 3, wherein the obtaining the digital information comprises generating a sequence representation of the image.

5. The computer-implemented system of claim 4, wherein the sequence representation comprises a property of the UIE, wherein the property comprises a class of the UIE, a bounding box of the UIE, a position of the UIE, a text in the UIE, or any combination thereof.

6. The computer-implemented system of any preceding claim, wherein the action identifies a target UIE.

7. The computer-implemented system of claim 6, wherein the target UIE is identified among a set of UIEs based on a spatial characteristic of the target UIE in relation to other user interface elements.

8. The computer-implemented system of claim 7, wherein the spatial characteristic comprises distance between the target UIE and the other UIEs in the set.

9. The computer-implemented system of claim 8, wherein the distance comprises a relative normalized pixel distance between the target UIE and the other UIEs in the set, optionally wherein the relative normalized pixel distance is stored in table for retrieval.

10. The computer-implemented system of any preceding claim, wherein the action comprises:

   clicking the target UIE; and/or
   a combination key press; and/or
   a loop, wherein the loop performs the action on an iterable data type.

11. The computer-implemented system of any preceding claim, wherein the task comprises:

   dictating; and/or
   searching; and/or
   ordering an item; and/or
   interacting with graphical user interface elements.

12. The computer-implemented system of any preceding claim, wherein the expression comprises natural language.

13. The computer-implemented system of any preceding, wherein the computer-executable program comprises instructions that are executable at a user-interface level.

14. The computer-implemented system of any preceding claim, wherein the computer-executable program comprises instructions that are executable above a firmware level, an operating system level, or an application programming interface level.

15. A computer-implemented system comprising: a digital processing device comprising: at least one processor, an operating system configured to perform executable instructions, a memory, and a computer program including instructions executable by the digital processing device to perform a computer task by a user expressing an expression to the computer-implemented system, and wherein the computer-implemented system is configured to perform the task by generating and executing a computer-executable program, comprising:

   (a) determining the computer task from the expression, by:

      i. receiving the user's expression through a receiver in electronic communication with the computer-implemented system; and
      ii. processing the user's expression using a large language model to generate a prediction of the computer task that is desired by the user based on the user's expression, wherein the computer task involves performing an action using an electronic medical record application, and wherein the prediction is non-deterministic;

   (b) determining a state of digital information displayed on a display in electronic communication with the computer-implemented system, by:

i. obtaining an image of a graphical user interface (GUI) that is shown on the display;

ii. detecting a plurality of user interface elements (UIEs) in the image;

iii. generating a sequence representation of the image comprising a plurality of properties of the UIEs; and

iv. matching, using a neural network, the sequence representation to a reference in a set of reference digital information;

(c) determining the action to be performed, by:

i. matching the computer task and the state to the action in a set of possible actions, wherein the set of possible actions are tabulated and indexed by possible computer tasks and possible states, and wherein the matching is deterministic; and

ii. identifying the target UIE among the plurality of UIEs in the digital information based on the action and a spatial characteristic of the target UIE in relation to other UIEs in the plurality, wherein the spatial characteristic comprises a relative normalized pixel distance between the target UIE and the other UIEs in the plurality; and

(d) generating and executing a computer-executable program that comprises instructions for performing the action, thereby performing the computer task.

FIG. 1

Obtain image of GUI — 203

Detect UIEs in the image — 204

Generate representation of the image with UIE properties — 205

Match representation of the image to representation of a reference image — 206

201 — Process user expression using a language model to predict user EMR task

Task — 202

State — 207

209 — Tabular Policy Database

Match task and state to an action — 208

ambiGUIty — 211

Identify target UIE — 210

Action

212 — Generate a computer-executable program for the action

213 — Execute computer-executable program

**FIG. 2**

**FIG. 3**

FIG. 4A      FIG. 4B      FIG. 4C

**FIG. 5**

LLM + memory
to compose, memorise and 'think' how to complete tasks

UIED and OCR AI
to see the screen and interpret the buttons and fields.

Robotic automation
to take over the mouse and

- On device
- Detected all buttons and fields
- Reads the text with OCR
- Matches that to a memory of 'states'

Human-capable set of tasks = Turing-complete Action Language set of tasks

Turing-incomplete Action language (A)

A - click

A - click - keypress

A - click - keypress - ambiGUIty

# FIG. 6

700

**Computer System**

740

720 / 730

**Processor(s)** 701

702 Cache

Network Interface

Network

721

Graphics Control

703 **Memory**

704 RAM
705 ROM
706 BIOS

Video Interface 722

Display 732

Storage Control 707

Input Interface 723

Input Device(s) 734

708 **Storage**

Operating System

710 / 709

Exec

Output Interface 724

Output Device(s) 733

Storage Device Interface 725

Storage Device(s) 735

712 Data 711

API Applications

Storage Medium Interface 726

Storage Media 736

**FIG. 7**

Europäisches
Patentamt
European
Patent Office
Office européen
des brevets

# EUROPEAN SEARCH REPORT

**Application Number**

EP 25 20 1081

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 11 355 119 B2 (BOLA TECH INC [US]) 7 June 2022 (2022-06-07) * Fig. 1; Fig. 2; Fig. 3; Fig. 4A; claim 1; col 1 lines 41-48; col 3 lines 6-36; col 3 lines 40-43; col 4 lines 13-17; col 4 lines 21-24; col 5 line 58 - col 6 line 2; col 6 lines 54-62; col 8 lines 3-7; col 8 lines 8-21; col 8 lines 34-42; col 8 lines 56 - col 9 line 23; col 9 lines 41-45; col 10 lines 33-59 * ----- | 1-15 | INV. G16H40/63 ADD. G16H10/60 |
| A,P | US 2024/320247 A1 (CHEN WEN-SHYEN [TW] ET AL) 26 September 2024 (2024-09-26) * para 6, 10, 11, 17, 21, 22, 32 * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 26 January 2026 | Bankwitz, Robert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...........................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 20 1081

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2026

| Patent document cited in search report | | Publication date | Patent family member(s) | | Publication date |
|---|---|---|---|---|---|
| US 11355119 | B2 | 07-06-2022 | CA | 3125845 A1 | 24-01-2022 |
| | | | US | 2022028382 A1 | 27-01-2022 |
| | | | US | 2022375471 A1 | 24-11-2022 |
| US 2024320247 | A1 | 26-09-2024 | NONE | | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 83090824